# EUROPEAN PATENT APPLICATION

(11) **EP 4 813 407 A1**
(43) Date of publication of application: **30.09.2026**
(21) Application number: 24893592.6
(22) Date of filing: 22.11.2024
(51) Int. Cl.: A61K 39/395, C07K 16/28, A61K 47/68, A61K 47/65, A61P 35/00

(54) **METHOD FOR TREATING TUMORS WITH ANTI-HER3 ANTIBODY DRUG CONJUGATE AND ANTI-VEGF ANTIBODY**

(30) Priority: 24.11.2023 CN 202311580719
(71) Applicant: Jiangsu Hengrui Pharmaceuticals Co., Ltd., Lianyungang, Jiangsu 222047 (CN)
(72) Inventor: SHI, Wei, Lianyungang, Jiangsu 222047 (CN)
(74) Representative: dompatent
(86) International application number: PCT/CN2024/133856
(87) International publication number: WO 2025/108438

(57) **Abstract**

A method for treat tumors with an anti-HER3 antibody drug conjugate and an anti-VEGF antibody. In particular, the present invention relates to the use of an anti-HER3 antibody drug conjugate in combination with a VEGF signaling pathway inhibitor in the preparation of a drug for treating tumors and the use of a combination of an anti-HER3 antibody drug conjugate and a VEGF signaling pathway inhibitor in the preparation of a drug for treating tumors.

## Description

The present application claims priority to Chinese Patent Application No. 202311580719.2 filed on November 24, 2023, which is incorporated herein by reference in its entirety.

### TECHNICAL FIELD

The present disclosure pertains to the field of pharmaceutics and relates to a method for treating a tumor with an anti-HER3 antibody-drug conjugate and an anti-VEGF antibody and pharmaceutical use.

### BACKGROUND

HER3 (epidermal growth factor receptor 3, ErbB-3 or HER3) is a member of the epidermal growth factor receptor (EGFR) family. This family includes HER1 (erbB1, EGFR), HER2 (erbB2, NEU), HER3 (erbB3), and HER4 (erbB4). These receptors each comprise 3 parts: an extracellular region, a transmembrane region, and an intracellular region. The extracellular region comprises 4 domains. The intracellular region comprises 1 intracellular tyrosine kinase domain for signaling and 1 tail in the cytoplasm comprising tyrosine phosphorylation residues. When ligands bind to extracellular domains I and III, cell signaling is initiated. Normally, these receptors mediate cell division, migration, survival, and organ development. When the EGFR family members mutate, the resulting aberrant signaling stimulates cell survival, which is associated with cancer progression. The basic principle behind the activation and physiological action of the HER3 receptor is similar to those of the other family members, except that its ligands include neuregulin 1 (NRG-1) and neuregulin 2 (NRG-2) and that activated HER3 cannot form a homomer but only a heterodimer with EGFR or HER2. In the process of HER3 forming a heterodimer, its intracellular domain exhibits high tyrosine phosphorylase activity. Structural analysis shows that the intracellular domain of HER3 has six P85 (PI-3K subunit) binding sites. This specific structure determines that HER3, when interacting with P85 regulatory subunits, can recruit up to six PI-3K to the regulatory subunit sites, thereby strongly activating the PI-3K signaling pathway. In fact, the HER3/HER2 dimer is the most active of the HER dimers. EGFRs are widely distributed on the surface of the cells such as mammalian epithelial cells, fibroblasts, glial cells, and keratinocytes. The EGFR signaling pathway plays an important role in physiological processes such as cell growth, proliferation, and differentiation.

An antibody-drug conjugate (ADC) is formed by covalently linking a cytotoxic small-molecule drug to an antibody via a chemical linker. Using the antibody as a carrier, it delivers the small-molecule drug to target cells. It combines the high targeting specificity of an antibody with the potent killing activity of a cytotoxic drug against target cells, thereby improving the efficacy of chemotherapy while reducing systemic exposure and toxicity. The endocytosis efficiency of the HER3 receptor is much higher than that of other members of the family, and thus the HER3 receptor is more suitable for being a target of ADC drugs.

Developing safe and effective clinical therapeutic strategies targeting HER3 is an important problem that needs to be solved in the art.

### SUMMARY

The present disclosure provides a method for treating a tumor with an anti-HER3 antibody-drug conjugate and pharmaceutical use.

In a first aspect, the present disclosure provides use of an anti-HER3 antibody-drug conjugate in combination with a VEGF signaling pathway inhibitor in the manufacture of a medicament for treating a tumor.

In a second aspect, the present disclosure provides use of a combination of an anti-HER3 antibody-drug conjugate and a VEGF signaling pathway inhibitor in the manufacture of a medicament for treating a tumor.

In a third aspect, the present disclosure provides use of a pharmaceutical composition in the manufacture of a medicament for treating a tumor, wherein the pharmaceutical composition comprises an anti-HER3 antibody-drug conjugate and a VEGF signaling pathway inhibitor.

In a fourth aspect, the present disclosure provides a method for treating a subject with a tumor, which comprises administering to the subject an anti-HER3 antibody-drug conjugate and a VEGF signaling pathway inhibitor.

In a fifth aspect, the present disclosure provides an anti-HER3 antibody-drug conjugate for use in treating a subject with a tumor, wherein the subject also receives treatment with a VEGF signaling pathway inhibitor.

In a sixth aspect, the present disclosure provides an anti-HER3 antibody-drug conjugate in combination with a VEGF signaling pathway inhibitor for use in treating a tumor.

In a seventh aspect, the present disclosure provides a VEGF signaling pathway inhibitor for use in treating a subject with a tumor, wherein an anti-HER3 antibody-drug conjugate is also administered to the subject.

In an eighth aspect, the present disclosure provides a pharmaceutical composition, which comprises an anti-HER3 antibody-drug conjugate and a VEGF signaling pathway inhibitor.

In some embodiments, as described in the first to seventh aspects above, the tumor is a solid tumor. In some embodiments, the tumor is non-small cell lung cancer. In some specific embodiments, the tumor is non-squamous cell non-small cell lung cancer. In some embodiments, the tumor includes, but is not limited to, any one or any combination of the following:
(1) a locally advanced or metastatic solid tumor (e.g., locally advanced or metastatic NSCLC);
(2) locally advanced or metastatic non-squamous cell carcinoma (e.g., locally advanced or metastatic non-squamous cell NSCLC);
(3) an advanced or metastatic solid tumor that has relapsed or progressed after standard treatment, or for which no standard treatment regimen exists, or that is not eligible for standard treatment at the current stage;
(4) an unresectable locally advanced or metastatic solid tumor that has relapsed or progressed after standard treatment, or for which no standard treatment regimen exists, or that is not eligible for standard treatment at the current stage;
(5) an EGFR-mutant tumor (e.g., EGFR-mutant NSCLC);
(6) a subject with the tumor has a history of treatment with a tyrosine kinase inhibitor (TKI); and
(7) a tumor that has failed EGFR-TKI therapy (e.g., NSCLC that has failed EGFR-TKI therapy). In some embodiments, "locally advanced or metastatic NSCLC" refers to a subject with histologically or cytologically confirmed locally advanced or metastatic non-small cell lung cancer. In some specific embodiments, "locally advanced or metastatic NSCLC" refers to a subject with histologically or cytologically confirmed unresectable locally advanced or metastatic non-small cell lung cancer. Illustratively, the histological or cytological confirmation results are based on treatment guidelines such as CSCO, NCCN, and ESMO.

In some specific embodiments, "locally advanced or metastatic NSCLC" refers to a subject with histologically or cytologically confirmed unresectable locally advanced or metastatic non-squamous cell non-small cell lung cancer.

In some embodiments, the "EGFR mutation" and "EGFR mutation-positive" are used interchangeably and may be sensitive mutations or non-sensitive mutations. In some embodiments, the " EGFR mutation" is an exon 19 deletion (19del) and an exon 21L858R mutation (L858R), or other mutations (e.g., G719X or L861Q).

In the present disclosure, the standard treatment regimens are well known to those skilled in the art, e.g., treatment regimens disclosed in treatment guidelines approved by the supervision department. For example, the treatment regimens include, but are not limited to, the detailed latest information provided in the NCCN guidelines and the CSCO guidelines regarding standard treatment regimens for various cancers.

### Anti-HER3 antibody-drug conjugate

In some embodiments, the anti-HER3 antibody-drug conjugate is derived from an antibody-drug conjugate of any structure in WO2022078425A1 and WO2020063676A1, and the structures and the preparation methods of the immunoconjugates and other related contents in the above patent are incorporated herein by reference.

In some embodiments, the anti-HER3 antibody-drug conjugate has a structure shown as the formula below: 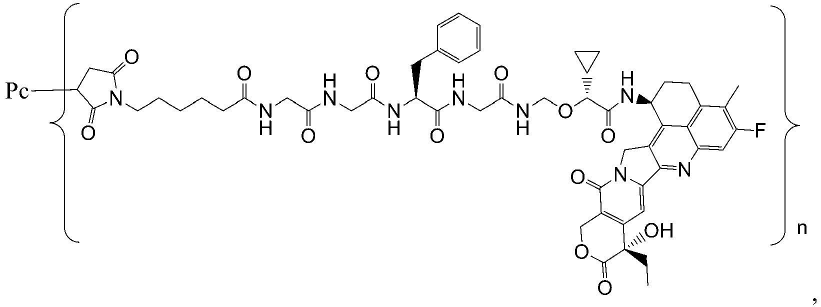 wherein n is 1-8 and is a decimal or an integer, and Pc is an anti-HER3 antibody.

In some embodiments, n is 4.0±0.4.

In the present disclosure, the term "antibody" is used in the broadest sense and encompasses a variety of antibody structures, including but not limited to monoclonal antibodies, polyclonal antibodies, multispecific antibodies (e.g., bispecific antibodies), full-length antibodies, or antigen-binding fragments thereof (also known as "antigen-binding moieties"), so long as they exhibit the desired antigen-binding activity.

In some embodiments, the anti-HER3 antibody is derived from an anti-HER3 antibody or an antigen-binding fragment thereof of any species in WO2022078425A1, and the antibody sequences, the preparation methods, and other related contents in the above patent are incorporated herein by reference.

In some embodiments, the anti-HER3 antibody comprises a heavy chain variable region (VH) and a light chain variable region (VL), wherein the heavy chain variable region comprises a HCDR1, a HCDR2, and a HCDR3 set forth in SEQ ID NO: 1, SEQ ID NO: 2, and SEQ ID NO: 3, respectively, and/or the light chain variable region comprises a LCDR1, a LCDR2, and a LCDR3 set forth in SEQ ID NO: 4, SEQ ID NO: 5, and SEQ ID NO: 6, respectively.

The CDR sequences described above are set forth in the table below:

**Table 1. CDR sequences obtained based on the Kabat numbering scheme**

| Antibody | HER3-29 | Sequence No. |
|---|---|---|
| Heavy chain CDR1 | DYAMH | SEQ ID NO: 1 |
| Heavy chain CDR2 | GISWNSGSIGYADSVKG | SEQ ID NO: 2 |
| Heavy chain CDR3 | EGLPGLDY | SEQ ID NO: 3 |
| Light chain CDR1 | RASQHVGTYLN | SEQ ID NO: 4 |
| Light chain CDR2 | GAANLQS | SEQ ID NO: 5 |
| Light chain CDR3 | QQSYNTPPFS | SEQ ID NO: 6 |

The CDRs are defined according to the Kabat numbering scheme.

In some embodiments, the anti-HER3 antibody comprises any 1, any 2, any 3, any 4, any 5, or 6 CDRs selected from the group consisting of a HCDR1, a HCDR2, a HCDR3, a LCDR1, a LCDR2, and a LCDR3 set forth in SEQ ID NO: 1 to SEQ ID NO: 6.

In some embodiments, the anti-HER3 antibody comprises a heavy chain variable region (VH) and a light chain variable region (VL), wherein the heavy chain variable region comprises a HCDR1, a HCDR2, and a HCDR3 set forth in SEQ ID NO: 7, and the light chain variable region comprises a LCDR1, a LCDR2, and a LCDR3 set forth in SEQ ID NO: 8. The CDRs are defined according to the Kabat, IMGT, Chothia, AbM, or Contact numbering scheme. In some specific embodiments, the CDRs are defined according to the Kabat numbering scheme.

In some embodiments, provided is use of an anti-HER3 antibody-drug conjugate in combination with an anti-VEGF antibody or an antigen-binding fragment thereof in the manufacture of a medicament for treating a tumor, wherein the VH of the anti-HER3 antibody comprises a HCDR1, a HCDR2, and a HCDR3 set forth in SEQ ID NOs: 1-3, respectively, and the VL of the anti-HER3 antibody or the antigen-binding fragment thereof comprises a LCDR1, a LCDR2, and a LCDR3 set forth in SEQ ID NOs: 4-6, respectively.

In some embodiments, the anti-HER3 antibody is a murine antibody, a chimeric antibody, a humanized antibody, a human antibody, or an antigen-binding fragment of any one of the foregoing. In some specific embodiments, the anti-HER3 antibody is selected from the group consisting of a humanized antibody or an antigen-binding fragment thereof.

In some embodiments, the VH of the anti-HER3 antibody comprises the amino acid sequence set forth in SEQ ID NO: 7 or an amino acid sequence having at least 80% sequence identity thereto, and the VL thereof comprises the amino acid sequence set forth in SEQ ID NO: 8 or an amino acid sequence having at least 80% sequence identity thereto.
The heavy chain variable region of HER3: 
QVQLVQSGGGLVQPGRSLRLSCAASGFTFDDYAMHWVRQAPGKGLEWVSGISWNSG
SIGYADSVKGRFTISRDNAKNSLYLQMNSLRAEDTALYYCAKEGLPGLDYWGQGTLVT
VSS
SEQ ID NO: 7
The light chain variable region of HER3:

DIQMTQSPSSLSASIGDRATITCRASQHVGTYLNWYQQKPGKTPKLLISGAANLQSGVP
SRFSGSGSGTDFTLTISSLQPEDFATYYCQQSYNTPPFSFGQGTKVEIK
SEQ ID NO: 8

In some embodiments, the anti-HER3 antibody comprises any 1 or 2 of the aforementioned VH and VL.

In some embodiments, the anti-HER3 antibody further comprises an antibody constant region. For example, the heavy chain constant region in the antibody constant region is selected from the group consisting of human IgG1, IgG2, IgG3, and IgG4 constant regions and variants thereof; the light chain constant region in the antibody constant region is selected from the group consisting of κ and λ chain constant regions of a human antibody and variants thereof.

The sequences of the light chain variable region and light chain constant region described above are combined to form a light chain sequence, and each heavy chain variable region is combined with a heavy chain constant region to form a heavy chain sequence. Exemplary humanized antibody sequences are shown below:
The heavy chain of HER3-29: 
QVQLVQSGGGLVQPGRSLRLSCAASGFTFDDYAMHWVRQAPGKGLEWVSGISWNSG
SIGYADSVKGRFTISRDNAKNSLYLQMNSLRAEDTALYYCAKEGLPGLDYWGQGTLVT
VSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSDGSFFL
YSKLTVDKSRWQQGNVFSCSVMHEALHNHYTQKSLSLSPGK
SEQ ID NO: 9
The light chain of HER3-29:

DIQMTQSPSSLSASIGDRATITCRASQHVGTYLNWYQQKPGKTPKLLISGAANLQSGVP
SRFSGSGSGTDFTLTISSLQPEDFATYYCQQSYNTPPFSFGQGTKVEIKRTVAAPSVFIFPP
SDEQLKSGTASVVCLLNNFYPREAKVQWKVDNALQSGNSQESVTEQDSKDSTYSLSS
TLTLSKADYEKHKVYACEVTHQGLSSPVTKSFNRGEC
SEQ ID NO: 10

In some embodiments, the anti-HER3 antibody comprises a heavy chain and a light chain, wherein the heavy chain variable region comprises the amino acid sequence set forth in SEQ ID NO: 9 or an amino acid sequence having at least 80% identity thereto, and the light chain variable region comprises the amino acid sequence set forth in SEQ ID NO: 10 or an amino acid sequence having at least 80% identity thereto.

In some embodiments, the anti-HER3 antibody comprises any 1 or 2 of the aforementioned heavy and light chains.

In some embodiments, provided is use of an anti-HER3 antibody-drug conjugate in combination with an anti-VEGF antibody in the manufacture of a medicament for treating a tumor, wherein the heavy chain of the anti-HER3 antibody comprises the amino acid sequence set forth in SEQ ID NO: 9, and the light chain thereof comprises the amino acid sequence set forth in SEQ ID NO: 10.

In the context of the present disclosure, "at least 80%" or "at least 90%" encompasses 80% or more, e.g., at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, and a numerical range between any two of these numbers.

In some embodiments, the anti-HER3 antibody-drug conjugate is administered at a dose of about 0.1 mg/kg to about 20 mg/kg, e.g., about 1.0 mg/kg to about 20 mg/kg, about 1.0 mg/kg to about 12 mg/kg, about 1.0 mg/kg to about 10.5 mg/kg, about 1.0 mg/kg to about 10 mg/kg, about 1.0 mg/kg to about 9 mg/kg, about 1.0 mg/kg to about 8.5 mg/kg, about 1.0 mg/kg to about 8.0 mg/kg, about 1.5 mg/kg to about 10.5 mg, about 1.5 mg/kg to about 9.0 mg/kg, about 1.5 mg/kg to about 8.5 mg/kg, about 1.5 mg/kg to about 8.0 mg/kg, about 1.5 mg/kg to about 7.5 mg/kg; about 3.0 mg/kg to about 9.0 mg/kg, about 4.5 mg/kg to about 9.0 mg/kg, about 6.0 mg/kg to about 9.0 mg/kg, about 7.5 mg/kg to about 9.0 mg/kg, about 3.0 mg/kg to about 8.5 mg/kg, about 4.5 mg/kg to about 8.5 mg/kg, about 6.0 mg/kg to about 8.5 mg/kg, about 7.5 mg/kg to about 8.5 mg/kg, about 3.0 mg/kg to about 8.0 mg/kg, about 4.5 mg/kg to about 8.0 mg/kg, about 6.0 mg/kg to about 8.0 mg/kg, about 7.5 mg/kg to about 8.0 mg/kg, about 3.0 mg/kg to about 7.5 mg/kg, or any range between these point values.

In some embodiments, the anti-HER3 antibody-drug conjugate is administered at a dose selected from the group consisting of about 1.0 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2.0 mg/kg, about 2.1 mg/kg, about 2.2 mg/kg, about 2.3 mg/kg, about 2.4 mg/kg, about 2.5 mg/kg, about 2.6 mg/kg, about 2.7 mg/kg, about 2.8 mg/kg, about 2.9 mg/kg, about 3.0 mg/kg, about 3.1 mg/kg, about 3.2 mg/kg, about 3.4 mg/kg, about 3.5 mg/kg, about 3.6 mg/kg, about 3.8 mg/kg, about 4.0 mg/kg, about 4.2 mg/kg, about 4.3 mg/kg, about 4.5 mg/kg, about 4.6 mg/kg, about 4.8 mg/kg, about 5.0 mg/kg, about 5.1 mg/kg, about 5.3 mg/kg, about 5.5 mg/kg, about 5.6 mg/kg, about 5.8 mg/kg, about 6.0 mg/kg, about 6.1 mg/kg, about 6.3 mg/kg, about 6.4 mg/kg, about 6.5 mg/kg, about 6.6 mg/kg, about 6.8 mg/kg, about 6.9 mg/kg, about 7.0 mg/kg, about 7.2 mg/kg, about 7.4 mg/kg, about 7.5 mg/kg, about 7.6 mg/kg, about 7.8 mg/kg, about 8.0 mg/kg, about 8.1 mg/kg, about 8.3 mg/kg, about 8.5 mg/kg, about 8.8 mg/kg, about 9.0 mg/kg, about 9.5 mg/kg, about 10.0 mg/kg, about 10.5 mg/kg, about 12 mg/kg, about 12.5 mg/kg, about 13 mg/kg, about 13.5 mg/kg, about 14 mg/kg, about 16 mg/kg, about 18 mg/kg, about 19 mg/kg, and about 20 mg/kg.

In some embodiments, the anti-HER3 antibody-drug conjugate is administered at a frequency of once every 1 week, once every 2 weeks, once every 3 weeks, once every 4 weeks, once every 6 weeks, once every 8 weeks, once every 10 weeks, or once every 12 weeks. In some specific embodiments, the anti-HER3 antibody-drug conjugate is administered at a frequency of once every 3 weeks.

In some embodiments, the administration regimen for the anti-HER3 antibody-drug conjugate is selected from the group consisting of any one of the following:
(a₁) administered at a dose of about 0.1 mg/kg to about 20 mg/kg and at a frequency of once every 1 week, once every 2 weeks, once every 3 weeks, once every 4 weeks, once every 6 weeks, once every 8 weeks, once every 10 weeks, or once every 12 weeks;
(a₂) administered at a dose of about 1.0 mg/kg to about 13.5 mg/kg and at a frequency selected from the group consisting of once every 1 week, once every 2 weeks, once every 3 weeks, once every 4 weeks, once every 6 weeks, once every 8 weeks, and once every 10 weeks;
(a₃) administered at a dose of about 1.0 mg/kg to about 10.5 mg/kg and at a frequency selected from the group consisting of once every 1 week, once every 2 weeks, once every 3 weeks, once every 4 weeks, once every 6 weeks, once every 8 weeks, and once every 10 weeks;
(a₄) administered at a dose of about 1.0 mg/kg to about 9.0 mg/kg and at a frequency selected from the group consisting of once every 1 week, once every 2 weeks, once every 3 weeks, once every 4 weeks, once every 6 weeks, once every 8 weeks, and once every 10 weeks;
(a₅) administered at a dose of about 1.0 mg/kg to about 8.5 mg/kg and at a frequency selected from the group consisting of once every 1 week, once every 2 weeks, once every 3 weeks, once every 4 weeks, once every 6 weeks, once every 8 weeks, and once every 10 weeks;
(a₆) administered at a dose of about 1.0 mg/kg to about 8.0 mg/kg and at a frequency selected from the group consisting of once every 1 week, once every 2 weeks, once every 3 weeks, once every 4 weeks, once every 6 weeks, once every 8 weeks, and once every 10 weeks;
(a₇) administered at a dose of about 1.5 mg/kg to about 10.5 mg/kg and at a frequency selected from the group consisting of once every 1 week, once every 2 weeks, once every 3 weeks, once every 4 weeks, once every 6 weeks, once every 8 weeks, and once every 10 weeks;
(a₈) administered at a dose of about 1.5 mg/kg to about 9.0 mg/kg and at a frequency selected from the group consisting of once every 1 week, once every 2 weeks, once every 3 weeks, once every 4 weeks, once every 6 weeks, once every 8 weeks, and once every 10 weeks;
(a₉) administered at a dose of about 1.5 mg/kg to about 8.5 mg/kg and at a frequency selected from the group consisting of once every 1 week, once every 2 weeks, once every 3 weeks, once every 4 weeks, once every 6 weeks, once every 8 weeks, and once every 10 weeks;
(a₁₀) administered at a dose of about 1.5 mg/kg to about 8.0 mg/kg and at a frequency selected from the group consisting of once every 1 week, once every 2 weeks, once every 3 weeks, once every 4 weeks, once every 6 weeks, once every 8 weeks, and once every 10 weeks;
(a₁₁) administered at a dose of about 2 mg/kg to about 9.0 mg/kg and at a frequency selected from the group consisting of once every 1 week, once every 2 weeks, once every 3 weeks, once every 4 weeks, once every 6 weeks, once every 8 weeks, and once every 10 weeks;
(a₁₂) administered at a dose of about 2 mg/kg to about 8.0 mg/kg and at a frequency selected from the group consisting of once every 1 week, once every 2 weeks, once every 3 weeks, once every 4 weeks, once every 6 weeks, once every 8 weeks, and once every 10 weeks;
(a₁₃) administered at a dose of about 4 mg/kg to about 9.0 mg/kg and at a frequency selected from the group consisting of once every 1 week, once every 2 weeks, once every 3 weeks, once every 4 weeks, once every 6 weeks, once every 8 weeks, and once every 10 weeks;
(a₁₄) administered at a dose of about 4 mg/kg to about 8.0 mg/kg and at a frequency selected from the group consisting of once every 1 week, once every 2 weeks, once every 3 weeks, once every 4 weeks, once every 6 weeks, once every 8 weeks, and once every 10 weeks;
(a₁₅) administered at a dose of about 5 mg/kg to about 9.0 mg/kg and at a frequency selected from the group consisting of once every 1 week, once every 2 weeks, once every 3 weeks, once every 4 weeks, once every 6 weeks, once every 8 weeks, and once every 10 weeks;
(a₁₆) administered at a dose of about 5 mg/kg to about 8.0 mg/kg and at a frequency selected from the group consisting of once every 1 week, once every 2 weeks, once every 3 weeks, once every 4 weeks, once every 6 weeks, once every 8 weeks, and once every 10 weeks;
(a₁₇) administered at a dose of about 6 mg/kg to about 9.0 mg/kg and at a frequency selected from the group consisting of once every 1 week, once every 2 weeks, once every 3 weeks, once every 4 weeks, once every 6 weeks, once every 8 weeks, and once every 10 weeks;
(a₁₈) administered at a dose of about 6 mg/kg to about 8.0 mg/kg and at a frequency selected from the group consisting of once every 1 week, once every 2 weeks, once every 3 weeks, once every 4 weeks, once every 6 weeks, once every 8 weeks, and once every 10 weeks;
(a₁₉) about 1.0 mg/kg, about 1.5 mg/kg, about 3.0 mg/kg, about 4.5 mg/kg, about 5.0 mg/kg, about 5.5 mg/kg, about 6.0 mg/kg, about 6.5 mg/kg, about 7 mg/kg, about 7.5 mg/kg, about 8.0 mg/kg, about 8.5 mg/kg, about 9.0 mg/kg, about 10.5 mg/kg, about 12 mg/kg, or about 13.5 mg/kg; administered at a frequency selected from the group consisting of once every 1 week, once every 2 weeks, once every 3 weeks, once every 4 weeks, once every 6 weeks, once every 8 weeks, and once every 10 weeks;
(a₂₀) the anti-HER3 antibody-drug conjugate is administered at a dose of about 1.5 mg/kg and at a frequency of once every 3 weeks;
(a₂₁) the anti-HER3 antibody-drug conjugate is administered at a dose of about 3.0 mg/kg and at a frequency of once every 3 weeks;
(a₂₂) the anti-HER3 antibody-drug conjugate is administered at a dose of about 4.5 mg/kg and at a frequency of once every 3 weeks;
(a₂₃) the anti-HER3 antibody-drug conjugate is administered at a dose of about 5.0 mg/kg and at a frequency of once every 3 weeks;
(a₂₄) the anti-HER3 antibody-drug conjugate is administered at a dose of about 5.5 mg/kg and at a frequency of once every 3 weeks;
(a₂₅) the anti-HER3 antibody-drug conjugate is administered at a dose of about 6.0 mg/kg and at a frequency of once every 3 weeks;
(a₂₆) the anti-HER3 antibody-drug conjugate is administered at a dose of about 7.0 mg/kg and at a frequency of once every 3 weeks;
(a₂₇) the anti-HER3 antibody-drug conjugate is administered at a dose of about 7.5 mg/kg and at a frequency of once every 3 weeks;
(a₂₈) the anti-HER3 antibody-drug conjugate is administered at a dose of about 8.0 mg/kg and at a frequency of once every 3 weeks;
(a₂₉) the anti-HER3 antibody-drug conjugate is administered at a dose of about 8.5 mg/kg and at a frequency of once every 3 weeks;
(a₃₀) the anti-HER3 antibody-drug conjugate is administered at a dose of about 9.0 mg/kg and at a frequency of once every 3 weeks.

In some specific embodiments, in the administration regimen according to any one of (a₁)-(a₁₉) described above, the anti-HER3 antibody-drug conjugate is administered at a frequency of once every 3 weeks.

### VEGF signaling pathway inhibitor

In some embodiments, the VEGF signaling pathway inhibitor includes a VEGF ligand inhibitor and a VEGF receptor inhibitor. In some embodiments, the VEGF signaling pathway inhibitor is an anti-VEGF antibody.

In the present disclosure, the term "antibody" is used in the broadest sense and encompasses a variety of antibody structures, including but not limited to monoclonal antibodies, polyclonal antibodies, multispecific antibodies (e.g., bispecific antibodies), full-length antibodies, or antigen-binding fragments thereof (also known as "antigen-binding moieties"), so long as they exhibit the desired antigen-binding activity.

In some embodiments, the anti-VEGF antibody comprises a heavy chain variable region (VH) and a light chain variable region (VL), wherein the heavy chain variable region comprises a HCDR1, a HCDR2, and a HCDR3 set forth in SEQ ID NOs: 11-13, respectively, and the light chain variable region comprises a LCDR1, a LCDR2, and a LCDR3 set forth in SEQ ID NOs: 14-16, respectively.

The CDR sequences described above are set forth in the table below:

**Table 2. CDR sequences of anti-VEGF antibody**

| Antibody | Sequence |
|---|---|
| HCDR1 | NYGMN (SEQ ID NO: 11) |
| HCDR2 | WINTYTGEPTYAADFKR (SEQ ID NO: 12) |
| HCDR3 | YPHYYGSSHWYFDV (SEQ ID NO: 13) |
| LCDR1 | SASQDISNYLN (SEQ ID NO: 14) |
| LCDR2 | FTSSLHS (SEQ ID NO: 15) |
| LCDR3 | QQYSTVPWT (SEQ ID NO: 16) |

The CDRs described above are defined according to the Kabat, IMGT, Chothia, AbM, or Contact definition scheme. In some specific embodiments, the CDRs are defined according to the Kabat definition scheme.

In some embodiments, the anti-VEGF antibody comprises a heavy chain variable region (VH) and a light chain variable region (VL), wherein the heavy chain variable region comprises a HCDR1, a HCDR2, and a HCDR3 set forth in SEQ ID NOs: 11-13, respectively, and the light chain variable region comprises a LCDR1, a LCDR2, and a LCDR3 set forth in SEQ ID NOs: 14-16, respectively.

In some embodiments, provided is use of an anti-HER3 antibody-drug conjugate in combination with an anti-VEGF antibody or an antigen-binding fragment thereof in the manufacture of a medicament for treating a tumor, wherein the VH of the anti-VEGF antibody comprises a HCDR1, a HCDR2, and a HCDR3 set forth in SEQ ID NOs: 11-13, respectively, and the VL of the anti-VEGF antibody or the antigen-binding fragment thereof comprises a LCDR1, a LCDR2, and a LCDR3 set forth in SEQ ID NOs: 14-16, respectively.

In some embodiments, the anti-VEGF antibody or antigen-binding fragment thereof is a chimeric, humanized, or fully human antibody or antigen-binding fragment thereof.

In some embodiments, in the anti-VEGF antibody or antigen-binding fragment thereof, the heavy chain variable region comprises the amino acid sequence set forth in SEQ ID NO: 17 or an amino acid sequence having at least 80% or 90% sequence identity thereto, and the light chain variable region comprises the amino acid sequence set forth in SEQ ID NO: 18 or an amino acid sequence having at least 80% or 90% sequence identity thereto.
Heavy chain variable region: 



SEQ ID NO: 17;
Light chain variable region:



SEQ ID NO: 18;

In some embodiments, the heavy chain variable region of the anti-VEGF antibody comprises the amino acid sequence set forth in SEQ ID NO: 17 or an amino acid sequence having at least 80% identity thereto, and the light chain variable region thereof comprises the amino acid sequence set forth in SEQ ID NO: 18 or an amino acid sequence having at least 80% identity thereto.

In some embodiments, the anti-VEGF antibody further comprises a heavy chain constant region and/or a light chain constant region. For example, the heavy chain constant region in the antibody constant region is selected from the group consisting of human IgG1, IgG2, IgG3, and IgG4 constant regions and variants thereof; the light chain constant region in the antibody constant region is selected from the group consisting of κ and λ chain constant regions of a human antibody and variants thereof.

In some embodiments, the anti-VEGF antibody comprises a heavy chain and a light chain, wherein the heavy chain comprises the amino acid sequence set forth in SEQ ID NO: 19 or an amino acid sequence having at least 80% sequence identity thereto, and/or the light chain comprises the amino acid sequence set forth in SEQ ID NO: 20 or an amino acid sequence having at least 80% identity thereto.

In some embodiments, the anti-VEGF antibody comprises a heavy chain having the amino acid sequence set forth in SEQ ID NO: 19 and a light chain having the amino acid sequence set forth in SEQ ID NO: 20.
The heavy chain sequence of the anti-VEGF antibody: 
EVQLVESGGGLVQPGGSLRLSCAASGYTFTNYGMNWVRQAPGKGLEWVGWINTYTG
EPTYAADFKRRFTFSLDTSKSTAYLQMNSLRAEDTAVYYCAKYPHYYGSSHWYFDVW
GQGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTHT
CPPCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKG
QPREPQVYTLPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGK
SEQ ID NO: 19;
The light chain sequence of the anti-VEGF antibody:

DIQMTQSPSSLSASVGDRVTITCSASQDISNYLNWYQQKPGKAPKVLIYFTSSLHSGVPS
RFSGSGSGTDFTLTISSLQPEDFATYYCQQYSTVPWTFGQGTKVEIKRTVAAPSVFIFPPS
DEQLKSGTASVVCLLNNFYPREAKVQWKVDNALQSGNSQESVTEQDSKDSTYSLSST
LTLSKADYEKHKVYACEVTHQGLSSPVTKSFNRGEC
SEQ ID NO: 20;

Note: the underlined portions are the variable region sequences of the antibody heavy or light chains and the non-underlined portions are the antibody constant region sequences.

In some embodiments, provided is use of an anti-HER3 antibody-drug conjugate in combination with an anti-VEGF antibody in the manufacture of a medicament for treating a tumor, wherein the heavy chain of the anti-VEGF antibody comprises the amino acid sequence set forth in SEQ ID NO: 19, and the light chain thereof comprises the amino acid sequence set forth in SEQ ID NO: 20.

In some embodiments, the anti-VEGF antibody is administered at a dose of about 1.0 mg/kg to about 30 mg/kg, e.g., about 2.0 mg/kg to about 25 mg/kg, about 2.0 mg/kg to about 20 mg/kg, about 2.0 mg/kg to about 19 mg/kg, about 2.0 mg/kg to about 18 mg/kg, about 2.0 mg/kg to about 17 mg/kg, about 2.0 mg/kg to about 16 mg/kg, about 2.0 mg/kg to about 15 mg/kg, about 3.0 mg/kg to about 25 mg/kg, about 3.0 mg/kg to about 20 mg/kg, about 3.0 mg/kg to about 19 mg/kg, about 3.0 mg/kg to about 18 mg/kg, about 3.0 mg/kg to about 17 mg/kg, about 3.0 mg/kg to about 16 mg/kg, about 3.0 mg/kg to about 15 mg/kg, about 4.0 mg/kg to about 25 mg/kg, about 4.0 mg/kg to about 20 mg/kg, about 4.0 mg/kg to about 19 mg/kg, about 4.0 mg/kg to about 18 mg/kg, about 4.0 mg/kg to about 17 mg/kg, about 4.0 mg/kg to about 16 mg/kg, about 4.0 mg/kg to about 15 mg/kg, about 5.0 mg/kg to about 25 mg/kg, about 5.0 mg/kg to about 20 mg/kg, about 5.0 mg/kg to about 19 mg/kg, about 5.0 mg/kg to about 18 mg/kg, about 5.0 mg/kg to about 17 mg/kg, about 5.0 mg/kg to about 16 mg/kg, about 5.0 mg/kg to about 15 mg/kg, about 6.0 mg/kg to about 25 mg/kg, about 6.0 mg/kg to about 20 mg/kg, about 6.0 mg/kg to about 19 mg/kg, about 6.0 mg/kg to about 18 mg/kg, about 6.0 mg/kg to about 17 mg/kg, about 6.0 mg/kg to about 16 mg/kg, about 6.0 mg/kg to about 15 mg/kg, about 7.0 mg/kg to about 20 mg/kg, about 7.0 mg/kg to about 19 mg/kg, about 7.0 mg/kg to about 18 mg/kg, about 7.0 mg/kg to about 17 mg/kg, about 7.0 mg/kg to about 16 mg/kg, about 7.0 mg/kg to about 15 mg/kg, about 7.5 mg/kg to about 20 mg/kg, about 7.5 mg/kg to about 19 mg/kg, about 7.5 mg/kg to about 18 mg/kg, about 7.5 mg/kg to about 17 mg/kg, about 7.5 mg/kg to about 16 mg/kg, about 7.5 mg/kg to about 15 mg/kg, or any range between these point values.

In some embodiments, the anti-VEGF antibody is administered at a dose selected from the group consisting of about 2.0 mg/kg, about 3.0 mg/kg, about 4.0 mg/kg, about 5.0 mg/kg, about 6.0 mg/kg, about 6.5 mg/kg, about 7.0 mg/kg, about 7.5 mg/kg, 8.0 mg/kg, 8.5 mg/kg, about 9.0 mg/kg, about 9.5 mg/kg, about 10.0 mg/kg, about 10.5 mg/kg, about 11.0 mg/kg, about 11.5 mg/kg, about 12.0 mg/kg, about 12.5 mg/kg, about 13.0 mg/kg, about 13.5 mg/kg, about 14.0 mg/kg, about 14.5 mg/kg, about 15.0 mg/kg, about 15.5 mg/kg, about 16.0 mg/kg, about 16.5 mg/kg, about 17.0 mg/kg, about 18.0 mg/kg, about 19.0 mg/kg, about 20.0 mg/kg, about 21.0 mg/kg, about 22.0 mg/kg, about 23.0 mg/kg, about 24.0 mg/kg, about 25.0 mg/kg, and about 30.0 mg/kg. In some embodiments, the anti-VEGF antibody is administered at a frequency of once every 1 week, once every 2 weeks, once every 3 weeks, once every 4 weeks, once every 6 weeks, once every 8 weeks, once every 10 weeks, or once every 12 weeks. In some specific embodiments, the anti-VEGF antibody is administered at a frequency of once every 3 weeks.

In some embodiments, the administration regimen for the anti-VEGF antibody is selected from the group consisting of any one of the following:
(c₁) the anti-VEGF antibody is administered at a dose of about 1.0 mg/kg to about 30 mg/kg and at a frequency of once every 1 week, once every 2 weeks, once every 3 weeks, once every 4 weeks, once every 6 weeks, once every 8 weeks, once every 10 weeks, or once every 12 weeks;
(c₂) the anti-VEGF antibody is administered at a dose of about 2.0 mg/kg to about 20 mg/kg and at a frequency of once every 1 week, once every 2 weeks, once every 3 weeks, once every 4 weeks, once every 6 weeks, once every 8 weeks, once every 10 weeks, or once every 12 weeks;
(c₃) the anti-VEGF antibody is administered at a dose of about 2.0 mg/kg to about 19 mg/kg and at a frequency of once every 1 week, once every 2 weeks, once every 3 weeks, once every 4 weeks, once every 6 weeks, once every 8 weeks, once every 10 weeks, or once every 12 weeks;
(c₄) the anti-VEGF antibody is administered at a dose of about 2.0 mg/kg to about 18 mg/kg and at a frequency of once every 1 week, once every 2 weeks, once every 3 weeks, once every 4 weeks, once every 6 weeks, once every 8 weeks, once every 10 weeks, or once every 12 weeks;
(c₅) the anti-VEGF antibody is administered at a dose of about 2.0 mg/kg to about 17 mg/kg and at a frequency of once every 1 week, once every 2 weeks, once every 3 weeks, once every 4 weeks, once every 6 weeks, once every 8 weeks, once every 10 weeks, or once every 12 weeks;
(c₆) the anti-VEGF antibody is administered at a dose of about 2.0 mg/kg to about 16 mg/kg and at a frequency of once every 1 week, once every 2 weeks, once every 3 weeks, once every 4 weeks, once every 6 weeks, once every 8 weeks, once every 10 weeks, or once every 12 weeks;
(c₇) the anti-VEGF antibody is administered at a dose of about 3.0 mg/kg to about 20 mg/kg and at a frequency of once every 1 week, once every 2 weeks, once every 3 weeks, once every 4 weeks, once every 6 weeks, once every 8 weeks, once every 10 weeks, or once every 12 weeks;
(c₈) the anti-VEGF antibody is administered at a dose of about 3.0 mg/kg to about 19 mg/kg and at a frequency of once every 1 week, once every 2 weeks, once every 3 weeks, once every 4 weeks, once every 6 weeks, once every 8 weeks, once every 10 weeks, or once every 12 weeks;
(c₉) the anti-VEGF antibody is administered at a dose of about 3.0 mg/kg to about 18 mg/kg and at a frequency of once every 1 week, once every 2 weeks, once every 3 weeks, once every 4 weeks, once every 6 weeks, once every 8 weeks, once every 10 weeks, or once every 12 weeks;
(c₁₀) the anti-VEGF antibody is administered at a dose of about 3.0 mg/kg to about 17 mg/kg and at a frequency of once every 1 week, once every 2 weeks, once every 3 weeks, once every 4 weeks, once every 6 weeks, once every 8 weeks, once every 10 weeks, or once every 12 weeks;
(c₁₁) the anti-VEGF antibody is administered at a dose of about 3.0 mg/kg to about 16 mg/kg and at a frequency of once every 1 week, once every 2 weeks, once every 3 weeks, once every 4 weeks, once every 6 weeks, once every 8 weeks, once every 10 weeks, or once every 12 weeks;
(c₁₂) the anti-VEGF antibody is administered at a dose of about 4.0 mg/kg to about 20 mg/kg and at a frequency of once every 1 week, once every 2 weeks, once every 3 weeks, once every 4 weeks, once every 6 weeks, once every 8 weeks, once every 10 weeks, or once every 12 weeks;
(c₁₃) the anti-VEGF antibody is administered at a dose of about 4.0 mg/kg to about 19 mg/kg and at a frequency of once every 1 week, once every 2 weeks, once every 3 weeks, once every 4 weeks, once every 6 weeks, once every 8 weeks, once every 10 weeks, or once every 12 weeks;
(c₁₄) the anti-VEGF antibody is administered at a dose of about 4.0 mg/kg to about 18 mg/kg and at a frequency of once every 1 week, once every 2 weeks, once every 3 weeks, once every 4 weeks, once every 6 weeks, once every 8 weeks, once every 10 weeks, or once every 12 weeks;
(c₁₅) the anti-VEGF antibody is administered at a dose of about 2.0 mg/kg, about 3.0 mg/kg, about 4.0 mg/kg, about 5.0 mg/kg, about 6.0 mg/kg, about 6.5 mg/kg, about 7.0 mg/kg, about 7.5 mg/kg, 8.0 mg/kg, 8.5 mg/kg, about 9.0 mg/kg, about 9.5 mg/kg, about 10.0 mg/kg, about 10.5 mg/kg, about 11.0 mg/kg, about 11.5 mg/kg, about 12.0 mg/kg, about 12.5 mg/kg, about 13.0 mg/kg, about 13.5 mg/kg, about 14.0 mg/kg, about 14.5 mg/kg, about 15.0 mg/kg, about 15.5 mg/kg, about 16.0 mg/kg, about 16.5 mg/kg, about 17.0 mg/kg, about 18.0 mg/kg, about 19.0 mg/kg, about 20.0 mg/kg, about 21.0 mg/kg, about 22.0 mg/kg, about 23.0 mg/kg, about 24.0 mg/kg, about 25.0 mg/kg, or about 30.0 mg/kg, and at a frequency selected from the group consisting of once every 1 week, once every 2 weeks, once every 3 weeks, once every 4 weeks, once every 6 weeks, once every 8 weeks, once every 10 weeks, and once every 12 weeks;
(c₁₆) the anti-VEGF antibody is administered at a dose of about 6.0 mg/kg and at a frequency of once every 3 weeks;
(c₁₇) the anti-VEGF antibody is administered at a dose of about 6.5 mg/kg and at a frequency of once every 3 weeks;
(c₁₈) the anti-VEGF antibody is administered at a dose of about 7.0 mg/kg and at a frequency of once every 3 weeks;
(c₁₉) the anti-VEGF antibody is administered at a dose of about 7.5 mg/kg and at a frequency of once every 3 weeks;
(c₂₀) the anti-VEGF antibody is administered at a dose of about 8.0 mg/kg and at a frequency of once every 3 weeks;
(c₂₁) the anti-VEGF antibody is administered at a dose of about 8.5 mg/kg and at a frequency of once every 3 weeks;
(c₂₂) the anti-VEGF antibody is administered at a dose of about 9.0 mg/kg and at a frequency of once every 3 weeks;
(c₂₃) the anti-VEGF antibody is administered at a dose of about 9.5 mg/kg and at a frequency of once every 3 weeks;
(c₂₄) the anti-VEGF antibody is administered at a dose of about 10.0 mg/kg and at a frequency of once every 3 weeks;
(c₂₅) the anti-VEGF antibody is administered at a dose of about 10.5 mg/kg and at a frequency of once every 3 weeks;
(c₂₆) the anti-VEGF antibody is administered at a dose of about 11.0 mg/kg and at a frequency of once every 3 weeks;
(c₂₇) the anti-VEGF antibody is administered at a dose of about 11.5 mg/kg and at a frequency of once every 3 weeks;
(c₂₈) the anti-VEGF antibody is administered at a dose of about 12.0 mg/kg and at a frequency of once every 3 weeks;
(c₂₉) the anti-VEGF antibody is administered at a dose of about 12.5 mg/kg and at a frequency of once every 3 weeks;
(c₃₀) the anti-VEGF antibody is administered at a dose of about 13.0 mg/kg and at a frequency of once every 3 weeks;
(c₃₁) the anti-VEGF antibody is administered at a dose of about 13.5 mg/kg and at a frequency of once every 3 weeks;
(c₃₂) the anti-VEGF antibody is administered at a dose of about 14.0 mg/kg and at a frequency of once every 3 weeks;
(c₃₃) the anti-VEGF antibody is administered at a dose of about 14.5 mg/kg and at a frequency of once every 3 weeks;
(c₃₄) the anti-VEGF antibody is administered at a dose of about 15.0 mg/kg and at a frequency of once every 3 weeks;
(c₃₅) the anti-VEGF antibody is administered at a dose of about 15.5 mg/kg and at a frequency of once every 3 weeks;
(c₃₆) the anti-VEGF antibody is administered at a dose of about 16.0 mg/kg and at a frequency of once every 3 weeks;
(c₃₇) the anti-VEGF antibody is administered at a dose of about 17.0 mg/kg and at a frequency of once every 3 weeks;
(c₃₈) the anti-VEGF antibody is administered at a dose of about 18.0 mg/kg and at a frequency of once every 3 weeks.

In some specific embodiments, in the administration regimen according to any one of (c₁)-(c₁₅) described above, the anti-VEGF antibody is administered at a frequency of once every 3 weeks. In some embodiments, the administration regimen of the anti-HER3 antibody-drug conjugate in combination with the anti-VEGF antibody is selected from the group consisting of any one of the following:
(a₁) the anti-HER3 antibody-drug conjugate is administered at a dose of about 0.1 mg/kg to about 20 mg/kg and at a frequency of once every 1 week, once every 2 weeks, once every 3 weeks, once every 4 weeks, once every 6 weeks, once every 8 weeks, once every 10 weeks, or once every 12 weeks; and (c₁) the anti-VEGF antibody is administered at a dose of about 1.0 mg/kg to about 30 mg/kg and at a frequency of once every 1 week, once every 2 weeks, once every 3 weeks, once every 4 weeks, once every 6 weeks, once every 8 weeks, once every 10 weeks, or once every 12 weeks;
(a₂) administered at a dose of about 1.0 mg/kg to about 13.5 mg/kg and at a frequency selected from the group consisting of once every 1 week, once every 2 weeks, once every 3 weeks, once every 4 weeks, once every 6 weeks, once every 8 weeks, and once every 10 weeks; and (c₂) the anti-VEGF antibody is administered at a dose of about 2.0 mg/kg to about 20 mg/kg and at a frequency of once every 1 week, once every 2 weeks, once every 3 weeks, once every 4 weeks, once every 6 weeks, once every 8 weeks, once every 10 weeks, or once every 12 weeks;
(a₃) administered at a dose of about 1.0 mg/kg to about 10.5 mg/kg and at a frequency selected from the group consisting of once every 1 week, once every 2 weeks, once every 3 weeks, once every 4 weeks, once every 6 weeks, once every 8 weeks, and once every 10 weeks; and (c₃) the anti-VEGF antibody is administered at a dose of about 0.1 mg/kg to about 3 mg/kg and at a frequency of once every 1 week, once every 2 weeks, once every 3 weeks, once every 4 weeks, once every 6 weeks, once every 8 weeks, once every 10 weeks, or once every 12 weeks;
(a₄) administered at a dose of about 1.0 mg/kg to about 9.0 mg/kg and at a frequency selected from the group consisting of once every 1 week, once every 2 weeks, once every 3 weeks, once every 4 weeks, once every 6 weeks, once every 8 weeks, and once every 10 weeks; and (c₃) the anti-VEGF antibody is administered at a dose of about 2.0 mg/kg to about 19 mg/kg and at a frequency of once every 1 week, once every 2 weeks, once every 3 weeks, once every 4 weeks, once every 6 weeks, once every 8 weeks, once every 10 weeks, or once every 12 weeks;
(a₅) administered at a dose of about 1.0 mg/kg to about 8.5 mg/kg and at a frequency selected from the group consisting of once every 1 week, once every 2 weeks, once every 3 weeks, once every 4 weeks, once every 6 weeks, once every 8 weeks, and once every 10 weeks; and (c₃) the anti-VEGF antibody is administered at a dose of about 2.0 mg/kg to about 19 mg/kg and at a frequency of once every 1 week, once every 2 weeks, once every 3 weeks, once every 4 weeks, once every 6 weeks, once every 8 weeks, once every 10 weeks, or once every 12 weeks;
(a₆) administered at a dose of about 1.0 mg/kg to about 8.0 mg/kg and at a frequency selected from the group consisting of once every 1 week, once every 2 weeks, once every 3 weeks, once every 4 weeks, once every 6 weeks, once every 8 weeks, and once every 10 weeks; and (c₃) the anti-VEGF antibody is administered at a dose of about 2.0 mg/kg to about 19 mg/kg and at a frequency of once every 1 week, once every 2 weeks, once every 3 weeks, once every 4 weeks, once every 6 weeks, once every 8 weeks, once every 10 weeks, or once every 12 weeks;
(a₇) administered at a dose of about 1.5 mg/kg to about 10.5 mg/kg, and at a frequency selected from the group consisting of once every 1 week, once every 2 weeks, once every 3 weeks, once every 4 weeks, once every 6 weeks, once every 8 weeks, and once every 10 weeks; and (c₃) the anti-VEGF antibody is administered at a dose of about 2.0 mg/kg to about 19 mg/kg and at a frequency of once every 1 week, once every 2 weeks, once every 3 weeks, once every 4 weeks, once every 6 weeks, once every 8 weeks, once every 10 weeks, or once every 12 weeks;
(a₈) administered at a dose of about 1.5 mg/kg to about 9.0 mg/kg and at a frequency selected from the group consisting of once every 1 week, once every 2 weeks, once every 3 weeks, once every 4 weeks, once every 6 weeks, once every 8 weeks, and once every 10 weeks; and (c₃) the anti-VEGF antibody is administered at a dose of about 2.0 mg/kg to about 19 mg/kg and at a frequency of once every 1 week, once every 2 weeks, once every 3 weeks, once every 4 weeks, once every 6 weeks, once every 8 weeks, once every 10 weeks, or once every 12 weeks;
(a₉) administered at a dose of about 1.5 mg/kg to about 8.5 mg/kg and at a frequency selected from the group consisting of once every 1 week, once every 2 weeks, once every 3 weeks, once every 4 weeks, once every 6 weeks, once every 8 weeks, and once every 10 weeks; and (c₃) the anti-VEGF antibody is administered at a dose of about 2.0 mg/kg to about 19 mg/kg and at a frequency of once every 1 week, once every 2 weeks, once every 3 weeks, once every 4 weeks, once every 6 weeks, once every 8 weeks, once every 10 weeks, or once every 12 weeks;
(a₁₀) administered at a dose of about 1.5 mg/kg to about 8.0 mg/kg and at a frequency selected from the group consisting of once every 1 week, once every 2 weeks, once every 3 weeks, once every 4 weeks, once every 6 weeks, once every 8 weeks, and once every 10 weeks; and (c₃) the anti-VEGF antibody is administered at a dose of about 2.0 mg/kg to about 19 mg/kg and at a frequency of once every 1 week, once every 2 weeks, once every 3 weeks, once every 4 weeks, once every 6 weeks, once every 8 weeks, once every 10 weeks, or once every 12 weeks;
the anti-HER3 antibody-drug conjugate is administered at a dose of about 3.0 mg/kg and at a frequency of once every 3 weeks; and the anti-VEGF antibody is administered at a dose of 15.0 mg/kg and at a frequency of once every 3 weeks;
the anti-HER3 antibody-drug conjugate is administered at a dose of about 4.5 mg/kg and at a frequency of once every 3 weeks; and the anti-VEGF antibody is administered at a dose of 15.0 mg/kg and at a frequency of once every 3 weeks;
the anti-HER3 antibody-drug conjugate is administered at a dose of about 5.0 mg/kg and at a frequency of once every 3 weeks; and the anti-VEGF antibody is administered at a dose of 15.0 mg/kg and at a frequency of once every 3 weeks;
the anti-HER3 antibody-drug conjugate is administered at a dose of about 5.5 mg/kg and at a frequency of once every 3 weeks; and the anti-VEGF antibody is administered at a dose of 15.0 mg/kg and at a frequency of once every 3 weeks;
the anti-HER3 antibody-drug conjugate is administered at a dose of about 6.0 mg/kg and at a frequency of once every 3 weeks; and the anti-VEGF antibody is administered at a dose of 15.0 mg/kg and at a frequency of once every 3 weeks;
the anti-HER3 antibody-drug conjugate is administered at a dose of about 7.0 mg/kg and at a frequency of once every 3 weeks; and the anti-VEGF antibody is administered at a dose of 15.0 mg/kg and at a frequency of once every 3 weeks;
the anti-HER3 antibody-drug conjugate is administered at a dose of about 7.5 mg/kg and at a frequency of once every 3 weeks; and the anti-VEGF antibody is administered at a dose of 15.0 mg/kg and at a frequency of once every 3 weeks;
the anti-HER3 antibody-drug conjugate is administered at a dose of about 8.0 mg/kg and at a frequency of once every 3 weeks; and the anti-VEGF antibody is administered at a dose of 15.0 mg/kg and at a frequency of once every 3 weeks;
the anti-HER3 antibody-drug conjugate is administered at a dose of about 8.5 mg/kg and at a frequency of once every 3 weeks; and the anti-VEGF antibody is administered at a dose of 15.0 mg/kg and at a frequency of once every 3 weeks;
the anti-HER3 antibody-drug conjugate is administered at a dose of about 9.0 mg/kg and at a frequency of once every 3 weeks; and the anti-VEGF antibody is administered at a dose of 15.0 mg/kg and at a frequency of once every 3 weeks;
the anti-HER3 antibody-drug conjugate is administered at a dose of about 10.5 mg/kg and at a frequency of once every 3 weeks; and the anti-VEGF antibody is administered at a dose of 15.0 mg/kg and at a frequency of once every 3 weeks;
the anti-HER3 antibody-drug conjugate is administered at a dose of about 6.0 mg/kg and at a frequency of once every 3 weeks; and the anti-VEGF antibody is administered at a dose of 17.0 mg/kg and at a frequency of once every 3 weeks;
the anti-HER3 antibody-drug conjugate is administered at a dose of about 7.0 mg/kg and at a frequency of once every 3 weeks; and the anti-VEGF antibody is administered at a dose of 17.0 mg/kg and at a frequency of once every 3 weeks;
the anti-HER3 antibody-drug conjugate is administered at a dose of about 7.5 mg/kg and at a frequency of once every 3 weeks; and the anti-VEGF antibody is administered at a dose of 17.0 mg/kg and at a frequency of once every 3 weeks;
the anti-HER3 antibody-drug conjugate is administered at a dose of about 8.0 mg/kg and at a frequency of once every 3 weeks; and the anti-VEGF antibody is administered at a dose of 17.0 mg/kg and at a frequency of once every 3 weeks;
the anti-HER3 antibody-drug conjugate is administered at a dose of about 6.0 mg/kg and at a frequency of once every 3 weeks; and the anti-VEGF antibody is administered at a dose of 16.0 mg/kg and at a frequency of once every 3 weeks;
the anti-HER3 antibody-drug conjugate is administered at a dose of about 7.0 mg/kg and at a frequency of once every 3 weeks; and the anti-VEGF antibody is administered at a dose of 16.0 mg/kg and at a frequency of once every 3 weeks;
the anti-HER3 antibody-drug conjugate is administered at a dose of about 7.5 mg/kg and at a frequency of once every 3 weeks; and the anti-VEGF antibody is administered at a dose of 16.0 mg/kg and at a frequency of once every 3 weeks;
the anti-HER3 antibody-drug conjugate is administered at a dose of about 8.0 mg/kg and at a frequency of once every 3 weeks; and the anti-VEGF antibody is administered at a dose of 16.0 mg/kg and at a frequency of once every 3 weeks;
the anti-HER3 antibody-drug conjugate is administered at a dose of about 6.0 mg/kg and at a frequency of once every 3 weeks; and the anti-VEGF antibody is administered at a dose of 14.0 mg/kg and at a frequency of once every 3 weeks;
the anti-HER3 antibody-drug conjugate is administered at a dose of about 7.0 mg/kg and at a frequency of once every 3 weeks; and the anti-VEGF antibody is administered at a dose of 14.0 mg/kg and at a frequency of once every 3 weeks;
the anti-HER3 antibody-drug conjugate is administered at a dose of about 7.5 mg/kg and at a frequency of once every 3 weeks; and the anti-VEGF antibody is administered at a dose of 14.0 mg/kg and at a frequency of once every 3 weeks;
the anti-HER3 antibody-drug conjugate is administered at a dose of about 8.0 mg/kg and at a frequency of once every 3 weeks; and the anti-VEGF antibody is administered at a dose of 14.0 mg/kg and at a frequency of once every 3 weeks;
the anti-HER3 antibody-drug conjugate is administered at a dose of about 6.0 mg/kg and at a frequency of once every 3 weeks; and the anti-VEGF antibody is administered at a dose of 13.0 mg/kg and at a frequency of once every 3 weeks;
the anti-HER3 antibody-drug conjugate is administered at a dose of about 7.0 mg/kg and at a frequency of once every 3 weeks; and the anti-VEGF antibody is administered at a dose of 13.0 mg/kg and at a frequency of once every 3 weeks;
the anti-HER3 antibody-drug conjugate is administered at a dose of about 7.5 mg/kg and at a frequency of once every 3 weeks; and the anti-VEGF antibody is administered at a dose of 13.0 mg/kg and at a frequency of once every 3 weeks;
the anti-HER3 antibody-drug conjugate is administered at a dose of about 8.0 mg/kg and at a frequency of once every 3 weeks; and the anti-VEGF antibody is administered at a dose of 13.0 mg/kg and at a frequency of once every 3 weeks;
the anti-HER3 antibody-drug conjugate is administered at a dose of about 6.0 mg/kg and at a frequency of once every 3 weeks; and the anti-VEGF antibody is administered at a dose of 12.0 mg/kg and at a frequency of once every 3 weeks;
the anti-HER3 antibody-drug conjugate is administered at a dose of about 7.0 mg/kg and at a frequency of once every 3 weeks; and the anti-VEGF antibody is administered at a dose of 12.0 mg/kg and at a frequency of once every 3 weeks;
the anti-HER3 antibody-drug conjugate is administered at a dose of about 7.5 mg/kg and at a frequency of once every 3 weeks; and the anti-VEGF antibody is administered at a dose of 12.0 mg/kg and at a frequency of once every 3 weeks;
the anti-HER3 antibody-drug conjugate is administered at a dose of about 8.0 mg/kg and at a frequency of once every 3 weeks; and the anti-VEGF antibody is administered at a dose of 12.0 mg/kg and at a frequency of once every 3 weeks;
the anti-HER3 antibody-drug conjugate is administered at a dose of about 6.0 mg/kg and at a frequency of once every 3 weeks; and the anti-VEGF antibody is administered at a dose of 11.0 mg/kg and at a frequency of once every 3 weeks;
the anti-HER3 antibody-drug conjugate is administered at a dose of about 7.0 mg/kg and at a frequency of once every 3 weeks; and the anti-VEGF antibody is administered at a dose of 11.0 mg/kg and at a frequency of once every 3 weeks;
the anti-HER3 antibody-drug conjugate is administered at a dose of about 7.5 mg/kg and at a frequency of once every 3 weeks; and the anti-VEGF antibody is administered at a dose of 11.0 mg/kg and at a frequency of once every 3 weeks;
the anti-HER3 antibody-drug conjugate is administered at a dose of about 8.0 mg/kg and at a frequency of once every 3 weeks; and the anti-VEGF antibody is administered at a dose of 11.0 mg/kg and at a frequency of once every 3 weeks;
the anti-HER3 antibody-drug conjugate is administered at a dose of about 6.0 mg/kg and at a frequency of once every 3 weeks; and the anti-VEGF antibody is administered at a dose of 10.0 mg/kg and at a frequency of once every 3 weeks;
the anti-HER3 antibody-drug conjugate is administered at a dose of about 7.0 mg/kg and at a frequency of once every 3 weeks; and the anti-VEGF antibody is administered at a dose of 10.0 mg/kg and at a frequency of once every 3 weeks;
the anti-HER3 antibody-drug conjugate is administered at a dose of about 7.5 mg/kg and at a frequency of once every 3 weeks; and the anti-VEGF antibody is administered at a dose of 10.0 mg/kg and at a frequency of once every 3 weeks;
the anti-HER3 antibody-drug conjugate is administered at a dose of about 8.0 mg/kg and at a frequency of once every 3 weeks; and the anti-VEGF antibody is administered at a dose of 10.0 mg/kg and at a frequency of once every 3 weeks;
the anti-HER3 antibody-drug conjugate is administered at a dose of about 6.0 mg/kg and at a frequency of once every 3 weeks; and the anti-VEGF antibody is administered at a dose of 9.0 mg/kg and at a frequency of once every 3 weeks;
the anti-HER3 antibody-drug conjugate is administered at a dose of about 7.0 mg/kg and at a frequency of once every 3 weeks; and the anti-VEGF antibody is administered at a dose of 9.0 mg/kg and at a frequency of once every 3 weeks;
the anti-HER3 antibody-drug conjugate is administered at a dose of about 7.5 mg/kg and at a frequency of once every 3 weeks; and the anti-VEGF antibody is administered at a dose of 9.0 mg/kg and at a frequency of once every 3 weeks;
the anti-HER3 antibody-drug conjugate is administered at a dose of about 8.0 mg/kg and at a frequency of once every 3 weeks; and the anti-VEGF antibody is administered at a dose of 9.0 mg/kg and at a frequency of once every 3 weeks;
the anti-HER3 antibody-drug conjugate is administered at a dose of about 6.0 mg/kg and at a frequency of once every 3 weeks; and the anti-VEGF antibody is administered at a dose of 8.0 mg/kg and at a frequency of once every 3 weeks;
the anti-HER3 antibody-drug conjugate is administered at a dose of about 7.0 mg/kg and at a frequency of once every 3 weeks; and the anti-VEGF antibody is administered at a dose of 8.0 mg/kg and at a frequency of once every 3 weeks;
the anti-HER3 antibody-drug conjugate is administered at a dose of about 7.5 mg/kg and at a frequency of once every 3 weeks; and the anti-VEGF antibody is administered at a dose of 8.0 mg/kg and at a frequency of once every 3 weeks;
the anti-HER3 antibody-drug conjugate is administered at a dose of about 8.0 mg/kg and at a frequency of once every 3 weeks; and the anti-VEGF antibody is administered at a dose of 8.0 mg/kg and at a frequency of once every 3 weeks;
the anti-HER3 antibody-drug conjugate is administered at a dose of about 6.0 mg/kg and at a frequency of once every 3 weeks; and the anti-VEGF antibody is administered at a dose of 7.5 mg/kg and at a frequency of once every 3 weeks;
the anti-HER3 antibody-drug conjugate is administered at a dose of about 7.0 mg/kg and at a frequency of once every 3 weeks; and the anti-VEGF antibody is administered at a dose of 7.5 mg/kg and at a frequency of once every 3 weeks;
the anti-HER3 antibody-drug conjugate is administered at a dose of about 7.5 mg/kg and at a frequency of once every 3 weeks; and the anti-VEGF antibody is administered at a dose of 7.5 mg/kg and at a frequency of once every 3 weeks;
the anti-HER3 antibody-drug conjugate is administered at a dose of about 8.0 mg/kg and at a frequency of once every 3 weeks; and the anti-VEGF antibody is administered at a dose of 7.5 mg/kg and at a frequency of once every 3 weeks.

In some embodiments, the route of administration of the anti-VEGF antibody is oral administration, parenteral administration (including but not limited to intravenous injection, subcutaneous injection, and intramuscular injection), and percutaneous administration. In some specific embodiments, the route of administration of the anti-VEGF antibody is intravenous injection.

In some embodiments, the route of administration of the anti-HER3 antibody-drug conjugate is oral administration, parenteral administration (including but not limited to intravenous injection, subcutaneous injection, and intramuscular injection), and percutaneous administration. In some specific embodiments, the route of administration of the anti-HER3 antibody-drug conjugate is intravenous injection.

In some embodiments, the anti-HER3 antibody-drug conjugate is formulated into an injectable form. Illustratively, the injectable form of the anti-HER3 antibody-drug conjugate is an injectable solution or Freeze-dried powder comprising the anti-HER3 antibody-drug conjugate and one or more pharmaceutically acceptable auxiliary materials.

In some embodiments, the pharmaceutical composition comprising the anti-HER3 antibody-drug conjugate further comprises a buffer, a stabilizer, an osmotic pressure regulator, and/or a surfactant. Illustratively, the buffer is selected from the group consisting of any one of acetic acid-sodium acetate, succinic acid-sodium succinate, histidine-hydrochloride, and citric acid-sodium citrate buffers. In some specific embodiments, the buffer is acetic acid-sodium acetate. Illustratively, the surfactant may be selected from the group consisting of polysorbate (e.g., polysorbate 20 or polysorbate 80), poloxamer, Triton, sodium dodecyl sulfonate, sodium lauryl sulfonate, sodium octyl glycoside, lauryl-sulfobetaine, myristyl-sulfobetaine, linoleylsulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocamidopropyl-betaine, linoleamidopropyl-betaine, myristamidopropyl betaine, palmitamidopropyl betaine, isostearamidopropyl-betaine, myristamidopropyl-dimethylamine, palmitamidopropyl-dimethylamine, isostearamidopropyl-dimethylamine, sodium methyl cocoyl, sodium methyl oleyl taurate, polyethylene glycol, polypropylene glycol, copolymers of ethylene and propylene glycol, and the like. In some specific embodiments, the surfactant is a polysorbate. Illustratively, the surfactant is polysorbate 80 or polysorbate 20. In some specific embodiments, the surfactant is polysorbate 80. In some embodiments, the pharmaceutical composition comprising the anti-HER3 antibody-drug conjugate further comprises sugar. Illustratively, the "sugar" includes general compositions (CH₂O)ₙ and derivatives thereof, including monosaccharides, disaccharides, trisaccharides, polysaccharides, sugar alcohols, reducing sugars, non-reducing sugars, and the like. The sugar may be selected from the group consisting of glucose, sucrose, trehalose, α,α-trehalose dihydrate, lactose, fructose, maltose, dextran, glycerin, erythritol, glycerol, arabitol, sylitol, sorbitol, mannitol, melibiose, melezitose, raffinose, mannotriose, stachyose, maltose, lactulose, maltulose, glucitol, maltitol, lactitol, iso-maltulose, etc. In some specific embodiments, the "sugar" is sucrose.

In some embodiments, the anti-HER3 antibody-drug conjugate and the anti-VEGF antibody are administered in combination in the order of the anti-HER3 antibody-drug conjugate and the anti-VEGF antibody and at a required administration interval of, e.g., at least 30 min, at least 1 h, at least 2 h, etc.

In some embodiments, the anti-HER3 antibody-drug conjugate and the anti-VEGF antibody are administered in combination in the order of the anti-VEGF antibody and the anti-HER3 antibody-drug conjugate and at a required administration interval of, e.g., at least 30 min, at least 1 h, at least 2 h, etc.

### Terminology

In order to facilitate the understanding of the present disclosure, certain technical and scientific terms are specifically defined below. Unless otherwise specifically defined herein, all other technical and scientific terms used herein have the meanings generally understood by those of ordinary skill in the art to which the present disclosure pertains.

Unless the context clearly requires otherwise, throughout the description and the claims, the words "comprise", "have", "include", etc., are to be understood in an inclusive sense as opposed to an exclusive or exhaustive sense, that is to say, in the sense of "including but not limited to". "Optional" or "optionally" means that the event or circumstance subsequently described may, but does not necessarily, occur. This description includes the instance where the event or circumstance occurs or does not occur.

"About" or "approximately" means that a numerical value is within an acceptable margin of error for the specific value determined by those of ordinary skill in the art, and the numerical value depends in part on how the value is measured or determined (i.e., the limitations of the measurement system). For example, "about" may mean a standard deviation within 1 or greater than 1. Alternatively, "about" or "substantially comprise" may mean a range of at most 20%, e.g., a variation of between 1% and 15%, between 1% and 10%, between 1% and 5%, between 0.5% and 5%, or between 0.5% and 1%. In the present disclosure, every instance where a number or numerical range is preceded by the term "about" also includes embodiments of the given number. Unless otherwise specified, when a specific value is provided in the present application and claims, the meaning of "about" or "substantially comprise" should be assumed to be within an acceptable margin of error for that specific value.

The term "and/or", e.g., "X and/or Y", should be understood to mean "X and Y" or "X or Y" and should be used to provide explicit support for both meanings or either meaning.

In some embodiments, the present disclosure is defined as follows.

"Antibody-drug conjugate (ADC)" is formed by connecting an antibody or an antibody fragment to a cytotoxin with biological activity or a small-molecule drug with cell-killing activity via a stable chemical linker compound, in which case the binding specificity of the antibody for antigens that are either specific to tumor cells or highly expressed on them and the high efficiency of the cytotoxin are fully exploited, and toxic side effects on normal cells are avoided. Antibody-drug conjugates can bind to tumor cells precisely and reduce the impact on normal cells compared with conventional chemotherapeutic drugs.

If an antibody-drug conjugate shows no significant chemical change, then the antibody "retains its chemical stability" in a pharmaceutical formulation. Chemical stability can be assessed by detecting and quantifying proteins in chemically altered forms. Degradation processes that often change the chemical structure of proteins include hydrolysis or clipping (assessed by methods such as size exclusion chromatography and CE-SDS), oxidation (assessed by methods such as peptide mapping in combination with mass spectrometry or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.).

An antibody-drug conjugate "retains its biological activity" in a pharmaceutical formulation if the biological activity of the antibody-drug conjugate at a given time is within a predetermined range of the biological activity exhibited at the time the pharmaceutical formulation was prepared. The three-letter and single-letter codes for amino acids used in the present disclosure are as described in J. biol. chem., 243, p3558 (1968).

As used herein, the term "antibody" is used in the broadest sense and encompasses a variety of antibody structures, including but not limited to monoclonal antibodies, polyclonal antibodies, monospecific antibodies, multispecific antibodies (e.g., bispecific antibodies), full-length antibodies, and antibody fragments (or antigen-binding fragments, or antigen-binding moieties), so long as they exhibit the desired antigen-binding activity. An antibody may refer to an immunoglobulin, which is of a four-peptide-chain structure formed by two heavy chains and two light chains linked by interchain disulfide bonds. The heavy chain constant regions of immunoglobulins differ in amino acid composition and arrangement; therefore, they also differ in antigenicity. Accordingly, immunoglobulins can be divided into five classes, or isotypes of immunoglobulins, namely IgM, IgD, IgG, IgA, and IgE, and their corresponding heavy chains are µ chains, δ chains, γ chains, α chains, and ε chains, respectively. Igs of the same class can be divided into different subclasses based on the amino acid composition of their hinge regions and the number and positions of heavy chain disulfide bonds; for example, IgGs can be divided into IgG1, IgG2, IgG3, and IgG4. Light chains are divided into κ or λ chains based on their constant regions. Each of the five Ig classes may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus as follows: FR1, CDR1, FR2, CDR2, FR3, CDR3, and FR4. The 3 CDRs of the light chain refer to LCDR1, LCDR2, and LCDR3, and the 3 CDRs of the heavy chain refer to HCDR1, HCDR2, and HCDR3.

For the determination or definition of CDRs, the deterministic depiction of CDRs and identification of residues comprising binding sites of an antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol. 2018 Oct 16; 9: 2278).

The term "antigen-binding fragment" or "functional fragment" or "antigen-binding moiety" refers to one or more fragments of an intact antibody that retain the ability to specifically bind to an antigen. It is shown that a fragment of a full-length antibody can be used to perform the antigen-binding function of the antibody. Illustratively, examples of the binding fragment encompassed in the term "antigen-binding fragment" include (i) a Fab fragment, a monovalent fragment consisting of VL, VH, CL and CH1 domains; (ii) a F(ab')₂ fragment, a bivalent fragment comprising two Fab fragments linked by a disulfide bridge in the hinge region; (iii) an Fd fragment, consisting of VH and CH1 domains; (iv) an Fv fragment, consisting of VH and VL domains of a single arm of an antibody; (V) a dsFv, a stable antigen-binding fragment formed by VH and VL via interchain disulfide bonds; (vi) an scFv; (vii) a diabody, a bispecific antibody, and a multispecific antibody, comprising fragments such as an scFv, a dsFv, and a Fab.

The terms "specific binding", "selective binding", "selectively bind to", and "specifically bind to" refer to the binding of an antibody to an epitope on a predetermined antigen. Generally, the antibody binds with an affinity (KD) of less than about 10⁻⁸ M, e.g., less than about 10⁻⁹ M, 10⁻¹⁰ M, or 10⁻¹¹ M or less.

The term "KD" refers to the dissociation equilibrium constant for specific antibody-antigen interaction. Generally, the antibody of the present disclosure binds to IL-5 with a dissociation equilibrium constant (KD) of less than about 10⁻⁷ M, e.g., less than about 10⁻⁸ M, 10⁻⁹ M, or 10⁻¹⁰ M or less, e.g., as determined in a BIACORE instrument by the surface plasmon resonance (SPR) technique.

"Identity" refers to sequence similarity between two polynucleotide sequences or between two polypeptides. When positions in two sequences being compared are occupied by identical bases or amino acid monomer subunits, for example, if a position in each of two DNA molecules is occupied by adenine, the molecules are homologous at that position. The homology percentage between two sequences is a function of the number of matching or homologous positions shared by the two sequences divided by the number of positions compared × 100%. For example, in the optimal alignment of sequences, if 6 out of 10 positions of two sequences are matched or homologous, the two sequences are 60% homologous, and if 95 out of 100 positions of two sequences are matched or homologous, the two sequences are 95% homologous. Generally, two sequences, when aligned, are compared to give the maximum percent identity. For example, the comparison may be made by the BLAST algorithm, wherein the parameters of the algorithm are selected to give the maximum match between the reference sequences over the entire length of each sequence. The following references relate to the BLAST algorithm often used for sequence analysis: the BLAST algorithms: Altschul, S.F. et al., (1990) J. Mol. Biol., 215: 403-410; Gish, W., et al., (1993) Nature Genet., 3: 266-272; Madden, T.L. et al., (1996) Meth. Enzymol., 266: 131-141; Altschul, S.F. et al., (1997) Nucleic Acids Res., 25: 3389-3402; and Zhang, J. et al., (1997) Genome Res., 7: 649-656. Other conventional BLAST algorithms, such as one provided by NCBI BLAST, are also well known to those skilled in the art.

"Administer" and "treat", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids. "Administer" and "treat" may refer to, for example, therapeutic, pharmacokinetic, diagnostic, research, and experimental methods. Treatment of cells comprises contact between a reagent and cells, and contact between the reagent and a fluid, wherein the fluid is in contact with the cells. "Administer" and "treat" also refer to treating, e.g., cells, by reagents, diagnosis, binding compositions or by another cell *in vitro* and *ex vivo*. "Treat", when applied to human, veterinary, or research subjects, refers to therapeutic treatment, preventive or prophylactic measures, and research and diagnostic applications.

"Treatment" refers to administering a therapeutic agent, such as a composition comprising any one of the conjugation compounds of the present disclosure, either internally or externally to a patient with one or more disease symptoms on which the therapeutic agent is known to have a therapeutic effect. Typically, the therapeutic agent is administered in an amount effective to alleviate one or more disease symptoms in the patient or population being treated to induce regression of such symptoms or to inhibit the development of such symptoms to any clinically measurable degree. The amount of therapeutic agent effective to alleviate any specific disease symptom (also known as a "therapeutically effective amount") may vary depending on a variety of factors, such as the disease state, age, and weight of the patient, and the ability of the drug to produce a desired therapeutic effect in the patient. Whether a disease symptom has been palliated can be evaluated by any clinical test methods commonly used by doctors or other health care professionals to evaluate the severity or progression of the symptom. Although the embodiments of the present disclosure (for example, treatment methods or products) may not be effective in alleviating all the target disease symptoms in every patient, as determined according to any statistical testing methods known in the art, such as Student t-test, chi-square test, Mann and Whitney's U test, Kruskal-Wallis test (H test), Jonckheere-Terpstra test and Wilcoxon test, they should palliate the target disease symptoms in a statistically significant number of patients.

"Effective amount" comprises an amount sufficient to ameliorate or prevent a symptom or disorder of a medical disease. An effective amount also means an amount sufficient to allow or facilitate diagnosis. The effective amount for a particular patient or veterinary subject may vary depending on factors such as the disorder to be treated, the general health of the patient, the method and route and dose of administration, and the severity of side effects. An effective amount may be the maximum dose or administration regimen that avoids significant side effects or toxic effects.

The term "subject" or "patient" means a mammal, particularly a primate, and particularly a human being.

The "combination" described herein is a mode of administration and refers to the administration of at least one dose of an anti-HER3 antibody-drug conjugate and at least one dose of a VEGF signaling pathway inhibitor over a certain period of time, wherein both drugs exhibit pharmacological effects. The period of time may be within one administration cycle, such as within 4 weeks, within 3 weeks, within 2 weeks, within 1 week, within 24 h, or within 12 h. The anti-HER2 antibody-drug conjugate or the pharmaceutically acceptable salt thereof and the VEGF signaling pathway inhibitor may be administered simultaneously or sequentially. Such periods of time include treatment in which the anti-HER3 antibody-drug conjugate and the VEGF signaling pathway inhibitor are administered via the same route of administration or different routes of administration, or the anti-HER2 antibody-drug conjugate and the VEGF signaling pathway inhibitor are administered via the same route of administration or different routes of administration. The administration in combination described herein is selected from the group consisting of simultaneous administration, separate formulation and co-administration, and separate formulation and sequential administration.

The "n" in the anti-HER3 antibody-drug conjugate of the present disclosure refers to the average number of cytotoxic drugs loaded on each antibody or an antigen-binding fragment thereof in the antibody-drug conjugate molecule, and it may also be expressed as the ratio of the number of drugs to the number of antibodies, which is the average number of drugs per ADC molecule after a coupling reaction as identified by hydrophobic interaction chromatography (HIC) or mass spectrometry.

"Pharmaceutical composition" refers to a mixture containing one or more of the compounds described herein or physiologically/pharmaceutically acceptable salts or prodrugs thereof, and other chemical components, wherein the other components are, for example, physiologically/pharmaceutically acceptable carriers and excipients. The pharmaceutical composition is intended to promote administration to an organism and facilitate the absorption of the active ingredient so that it can exert its biological activity.

### DETAILED DESCRIPTION

The present disclosure is further described below with reference to examples, which, however, are not intended to limit the scope of the present disclosure. The experimental methods in the examples of the present disclosure in which specific conditions are not specified are generally performed under conventional conditions, for example, by referring to Antibodies: A Laboratory Manual and Molecular Cloning: A Laboratory Manual by Cold Spring Harbor Laboratory, or under conditions recommended by the manufacturers of the starting materials or commercial products. Reagents without specific sources indicated were commercially available conventional reagents.

### Example 1. Preparation of Anti-HER3 Antibody-Drug Conjugate

The anti-HER3 antibody-drug conjugates are Example 3-1 (n = 4.19), Example 3-2 (n = 2.91), Example 3-3 (n = 7.27) in WO2022078425A1 (incorporated herein by reference in its entirety), which have the following structure:

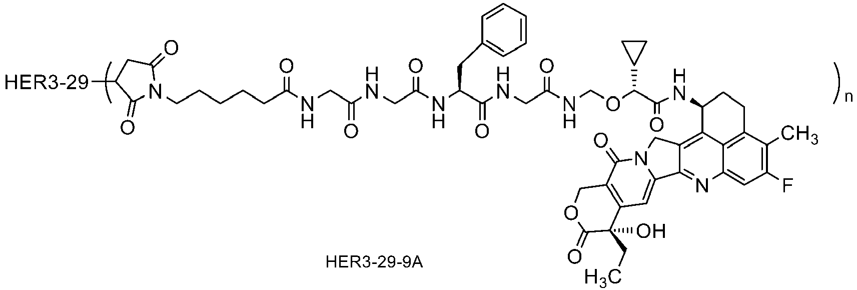

The structure of the anti-HER3 antibody-drug conjugate is abbreviated as the following structural formula ADC-1: 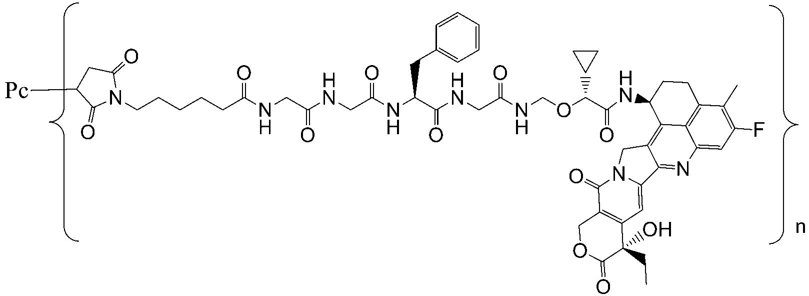 wherein n (DAR value) is about 4.0, which is equivalent to 4.0±0.4.

The HER3 antibody is derived from the antibody HER3-29 in WO2022078425A1, and the CDR sequences are shown in Table 1. The full-length heavy chain of antibody HER3-29 is set forth in SEQ ID NO: 9, and the full-length light chain thereof is set forth in SEQ ID NO: 10.

ADC-1 was prepared as an injection (sterile powder for injection) at a strength of 100 mg/vial.

### Example 2. Study on Safety, Tolerability, and Efficacy of ADC-1 for Injection in Combination with Anti-Tumor Therapy in Patients with Advanced Solid Tumors

### 1. Investigational drugs

1) ADC-1 prepared in Example 1.
2) Anti-VEGF antibody (bevacizumab). The heavy chain sequence is SEQ ID NO: 19, and the light chain sequence is SEQ ID NO: 20.

### 2. Enrolled subjects

1. Aged 18-75 years (inclusive), regardless of gender;
2) Part C1: subjects with histologically or cytologically confirmed unresectable locally advanced or metastatic non-squamous cell non-small cell lung cancer; Part C2: subjects with histologically or cytologically confirmed other locally advanced metastatic solid tumors;
3) Part C: relapse or progression after standard treatment, or no standard treatment regimen available, or not eligible for standard treatment at the current stage;
4) At least one measurable tumor lesion according to RECIST v1.1 (subjects with only non-target lesions are allowed to be enrolled in the phase IB);
5) All subjects enrolled must provide tumor tissue samples for HER3 expression and other assays (subjects who cannot provide tumor tissue samples are allowed to be enrolled in the phase IB). The tumor tissue samples should be neutral formalin-fixed, paraffin-embedded (FFPE) tissue blocks or at least 6 unstained tumor tissue sections, either fresh or archived, with fresh samples preferred. Subjects who are unable to provide freshly obtained tissue can provide tumor tissue samples that are archived within 12 months prior to the first study treatment. For subjects who cannot provide tumor tissue samples meeting the above requirements, discussion with the sponsor is required to determine whether to enroll;
6) ECOG physical strength score: 0-1;
7) Estimated survival time ≥ 12 weeks;
8. Having sufficient bone marrow and organ functions, with the following examinations required to be completed within 7 days prior to the first study treatment:
   a) blood routine examination (no blood transfusion or hematopoietic stimulating factor therapy within 14 days before the examination): neutrophil count (ANC) ≥ 1.5 × 10⁹/L (1,500/mm³), platelet count ≥ 100 × 10⁹/L (100,000/mm³), and hemoglobin (Hgb) ≥ 9.0 g/dL (90 g/L);
   b) liver function examination (no treatment with a liver-protecting drug has been received within 7 days before the tests): alanine aminotransferase (ALT) and aspartate aminotransferase (AST) ≤ 3 × ULN (for subjects with confirmed liver metastasis, ALT and AST ≤ 5 × ULN), and total bilirubin ≤ 1.5 × ULN (for subjects with confirmed Gilbert syndrome, total bilirubin ≤ 3 mg/dL);
   c) kidney function examination: serum creatinine ≤ 1.5 × ULN or creatinine clearance ≥ 60 mL/min (calculated using Cockcroft-Gault formula);
   d) Blood coagulation function examination: prothrombin time and partial thromboplastin time ≤ 1.5 × ULN.

### 3. Administration regimen

Part C is a clinical trial of ADC-1 in combination with bevacizumab for the treatment of locally advanced/metastatic non-squamous cell non-small cell lung cancer and other advanced solid tumors. The course of the study includes a screening period, a treatment period, and a follow-up period. The treatment period includes Phase IB and Phase II.

The study is divided into Part C1 and Part C2 according to the tumor types enrolled: Part C1 for locally advanced or metastatic non-squamous cell non-small cell lung cancer, and Part C2 for other locally advanced/metastatic solid tumors.

*Phase IB*: 3 dose groups are preset using an "i3 + 3" design for dose exploration:
Dose group 1: ADC-1 8.0 mg/kg Q3W in combination with bevacizumab 7.5 mg/kg Q3W;
Dose group 2: ADC-1 8.0 mg/kg Q3W in combination with bevacizumab 15 mg/kg Q3W;
Dose group 3: ADC-1 7.0 mg/kg Q3W in combination with bevacizumab 7.5 mg/kg Q3W.

Twenty-one days are counted as one treatment cycle, ADC-1 is administered by intravenous infusion (Q3W), and bevacizumab is administered by intravenous infusion (Q3W).

*Phase II*: The enrolled subjects receive the recommended dose of ADC-1 in combination with bevacizumab treatment until disease progression or until other criteria for treatment discontinuation are met.

### 4. Results

As of the data cutoff date, a total of 19 patients were enrolled. The details are shown in Table 2.

**Table 2. Condition of subjects**

| | | ADC-1 8.0 mg/kg + bevacizumab 7.5 mg/kg, N = 11 | ADC-1 8.0 mg/kg + bevacizumab 15 mg/kg, N=7* |
|---|---|---|---|
| Age, median (range), years | | 47 (39-72) | 58 (42-69) |
| Female, n (%) | | 9 (81.8) | 4 (57.1) |
| ECOG score, n (%) | 0 | 0 | 1 (14.3) |
| | 1 | 11 (100.0) | 6 (85.7) |
| Staging before enrollment, n (%)* | III | 1 (9.1) | 1 (14.3) |
| | IV | 10 (90.9) | 6 (85.7) |
| Pathological diagnosis, n (%) | Adenocarcinoma | 11 (100.0) | 7 (100.0) |
| Type of EGFR mutation | 19del | 6 (54.5) | 5 (71.4) |
| | L858R | 3 (27.3) | 2 (28.6) |
| | Other | 2 (18.2) | 0 |
| Prior lines of systemic anti-tumor therapy | Median | 1 (1-2) | 1 (1-2) |
| | ≥ 3 lines, n (%) | 0 | 0 |
| Prior third-generation EGFR-TKI, n (%) | | 9 (81.8) | 7 (100.0) |
| Prior platinum-containing chemotherapy, n (%) | | 0 | 0 |

| | | | |
|---|---|---|---|
| * Data entry for 2 subjects is pending due to delay. | | | |

### 2) Efficacy data

As can be seen from Table 3, at the highest dose, ADC-1 8.0 mg/kg in combination with bevacizumab 15 mg/kg resulted in an objective response rate (ORR) of 57.1% and a disease control rate (DCR) of 100.0%; ADC-1 8.0 mg/kg in combination with bevacizumab 7.5 mg/kg resulted in an ORR of 63.6% and a DCR of 100.0%. The combination regimen of ADC-1 and bevacizumab exhibits a good therapeutic effect.

**Table 3. Efficacy evaluation**

| | ADC-1 8.0 mg/kg + bevacizumab 7.5 mg/kg, N = 11 | ADC-1 8.0 mg/kg + bevacizumab 15 mg/kg, N=9* | Total, N = 20 |
|---|---|---|---|
| Subjects who have received at least one tumor efficacy evaluation, n | 11 | 7 | 18 |
| CR | 0 | 0 | 0 |
| PR | 7 (63.6) | 4 (57.1) | 11 (61.1) |
| SD | 4 (36.4) | 3 (42.9) | 7 (38.9) |
| PD | 0 | 0 | 0 |
| ORR,% (95% CI) | 63.6 | 57.1 | 61.1 |
| DCR,% (95% CI) | 100.0 | 100.0 | 100 |

| | | | |
|---|---|---|---|
| * Data entry for 2 subjects is pending due to delay. | | | |

### 3) Safety evaluation

In the ADC-1 8.0 mg/kg + bevacizumab 15 mg/kg group (N = 7), 2 cases (28.6%) reported TRAEs of grade ≥ 3, namely one case of decreased neutrophil count and one case of hypertension. In the ADC-1 8.0 mg/kg + bevacizumab 7.5 mg/kg group (N = 11), 7 cases (63.6%) reported treatment-related adverse events (TRAEs) of grade ≥ 3.

## Claims

1. Use shown in any one of the following (1)-(5):
(1) use of an anti-HER3 antibody-drug conjugate in combination with a VEGF signaling pathway inhibitor in the manufacture of a medicament for treating a tumor;
(2) use of a combination of an anti-HER3 antibody-drug conjugate and a VEGF signaling pathway inhibitor in the manufacture of a medicament for treating a tumor;
(3) use of a pharmaceutical composition in the manufacture of a medicament for treating a tumor, wherein the pharmaceutical composition comprises an anti-HER3 antibody-drug conjugate and a VEGF signaling pathway inhibitor;
(4) an anti-HER3 antibody-drug conjugate for use in treating a subject with a tumor, wherein a VEGF signaling pathway inhibitor is also administered to the subject; and
(5) a VEGF signaling pathway inhibitor for use in treating a subject with a tumor, wherein an anti-HER3 antibody-drug conjugate is also administered to the subject;
wherein the anti-HER3 antibody-drug conjugate has a structure shown as the formula below: 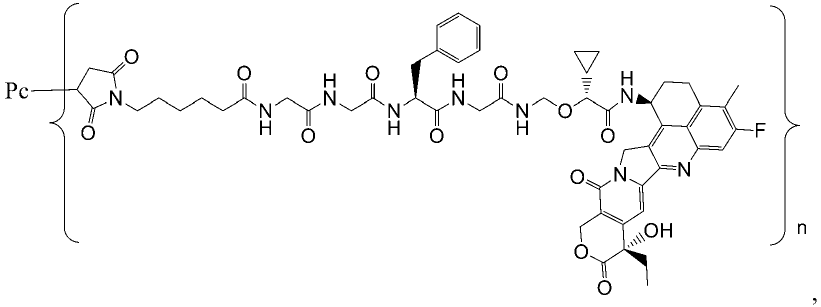
wherein n is 1-8, and Pc is an anti-HER3 antibody.

2. The use according to claim 1, wherein the tumor is non-small cell lung cancer, preferably non-squamous cell non-small cell lung cancer.

3. The use according to claim 2, wherein the tumor is selected from the group consisting of:
locally advanced or metastatic non-small cell lung cancer, or
locally advanced or metastatic non-squamous cell non-small cell lung cancer.

4. The use according to claim 1, wherein
(1) the tumor is an EGFR-mutant tumor, preferably an EGFR-mutant non-small cell lung cancer; and/or
(2) a subject with the tumor has a history of treatment with a tyrosine kinase inhibitor (TKI), preferably non-small cell lung cancer that has failed EGFR-TKI therapy;
preferably, the EGFR-mutant tumor is an EGFR-mutant tumor that has failed tyrosine kinase inhibitor (TKI) therapy.

5. The use according to any one of claims 1-4, wherein the VEGF signaling pathway inhibitor is an anti-VEGF antibody; preferably, the anti-VEGF antibody comprises a heavy chain variable region (VH) and a light chain variable region (VL), wherein the VH comprises a HCDR1, a HCDR2, and a HCDR3 set forth in SEQ ID NO: 11, SEQ ID NO: 12, and SEQ ID NO: 13, respectively, and the VL comprises a LCDR1, a LCDR2, and a LCDR3 set forth in SEQ ID NO: 14, SEQ ID NO: 15, and SEQ ID NO: 16, respectively.

6. The use according to claim 5, wherein the anti-VEGF antibody comprises a heavy chain variable region (VH) and a light chain variable region (VL), wherein the VH comprises the amino acid sequence set forth in SEQ ID NO: 17 or an amino acid sequence having at least 90% identity thereto, and the VL comprises the amino acid sequence set forth in SEQ ID NO: 18 or an amino acid sequence having at least 90% identity thereto;
preferably, the anti-VEGF antibody comprises a heavy chain and a light chain, wherein the heavy chain comprises the amino acid sequence set forth in SEQ ID NO: 19 or an amino acid sequence having at least 90% identity thereto, and the light chain comprises the amino acid sequence set forth in SEQ ID NO: 20 or an amino acid sequence having at least 90% identity thereto.

7. The use according to claim 5 or 6, wherein the anti-VEGF antibody is administered at a dose of about 1.0 mg/kg to about 30 mg/kg, about 2.0 mg/kg to about 25 mg/kg, about 2.0 mg/kg to about 20 mg/kg, about 2.0 mg/kg to about 19 mg/kg, about 2.0 mg/kg to about 18 mg/kg, about 2.0 mg/kg to about 17 mg/kg, or about 2.0 mg/kg to about 16 mg/kg,
preferably about 2.0 mg/kg, about 3.0 mg/kg, about 4.0 mg/kg, about 5.0 mg/kg, about 6.0 mg/kg, about 6.5 mg/kg, about 7.0 mg/kg, about 7.5 mg/kg, 8.0 mg/kg, 8.5 mg/kg, about 9.0 mg/kg, about 10.0 mg/kg, about 11.0 mg/kg, about 12.0 mg/kg, about 13.0 mg/kg, about 14.0 mg/kg, about 14.5 mg/kg, about 15.0 mg/kg, about 16.0 mg/kg, about 16.5 mg/kg, about 17.0 mg/kg, about 18.0 mg/kg, about 19.0 mg/kg, or about 20.0 mg/kg;
the anti-VEGF antibody is administered at a frequency of once every 1 week, once every 2 weeks, once every 3 weeks, once every 4 weeks, once every 6 weeks, once every 8 weeks, once every 10 weeks, or once every 12 weeks, preferably once every 3 weeks.

8. The use according to any one of claims 1-7, wherein the anti-HER3 antibody comprises a heavy chain variable region (VH) and a light chain variable region (VL), wherein the VH comprises a HCDR1, a HCDR2, and a HCDR3 set forth in SEQ ID NO: 1, SEQ ID NO: 2, and SEQ ID NO: 3, respectively, and the VL comprises a LCDR1, a LCDR2, and a LCDR3 set forth in SEQ ID NO: 4, SEQ ID NO: 5, and SEQ ID NO: 6, respectively;
preferably, the VH comprises the amino acid sequence set forth in SEQ ID NO: 7 or an amino acid sequence having at least 90% identity thereto, and the VL comprises the amino acid sequence set forth in SEQ ID NO: 8 or an amino acid sequence having at least 90% identity thereto;
more preferably, the anti-HER3 antibody comprises a heavy chain and a light chain, wherein the heavy chain comprises the amino acid sequence set forth in SEQ ID NO: 9 or an amino acid sequence having at least 90% identity thereto, and the light chain comprises the amino acid sequence set forth in SEQ ID NO: 10 or an amino acid sequence having at least 90% identity thereto.

9. The use according to any one of claims 1-8, wherein the anti-HER3 antibody-drug conjugate is administered at a dose of about 0.1 mg/kg to about 20 mg/kg, about 1.0 mg/kg to about 20 mg/kg, about 1.0 mg/kg to about 13.5 mg/kg, about 1.0 mg/kg to about 12 mg/kg, about 1.5 mg/kg to about 10.5 mg, about 1.5 mg/kg to about 9.0 mg/kg, about 1.5 mg/kg to about 8.5 mg/kg, or about 1.5 mg/kg to about 8.0 mg/kg,
preferably about 1.0 mg/kg, about 1.5 mg/kg, about 3.0 mg/kg, about 4.5 mg/kg, about 5.0 mg/kg, about 5.5 mg/kg, about 6.0 mg/kg, about 6.5 mg/kg, about 7.0 mg/kg, about 7.5 mg/kg, about 8.0 mg/kg, about 8.5 mg/kg, about 9.0 mg/kg, about 10.5 mg/kg, about 12 mg/kg, or about 13.5 mg/kg;
the anti-HER3 antibody-drug conjugate is administered at a frequency of once every 1 week, once every 2 weeks, once every 3 weeks, once every 4 weeks, once every 6 weeks, once every 8 weeks, once every 10 weeks, or once every 12 weeks, preferably once every 2 weeks or once every 3 weeks.

10. The use according to any one of claims 1-9, wherein
the anti-HER3 antibody-drug conjugate is administered at a dose of about 0.1 mg/kg to about 20 mg/kg and at a frequency of once every 1 week, once every 2 weeks, once every 3 weeks, once every 4 weeks, once every 6 weeks, once every 8 weeks, once every 10 weeks, or once every 12 weeks; and/or
the VEGF signaling pathway inhibitor is administered at a dose of about 1.0 mg/kg to about 30 mg/kg and at a frequency of once every 1 week, once every 2 weeks, once every 3 weeks, once every 4 weeks, once every 6 weeks, once every 8 weeks, once every 10 weeks, or once every 12 weeks;
preferably,
the anti-HER3 antibody-drug conjugate is administered at a dose of about 1.5 mg/kg to about 9.0 mg/kg, and the anti-HER3 antibody-drug conjugate is administered at a frequency of once every 3 weeks; and the VEGF signaling pathway inhibitor is administered at a dose of about 2 mg/kg to about 20 mg/kg and at a frequency of once every 3 weeks.

11. A method for treating a subject with a tumor, comprising administering to the subject an anti-HER3 antibody-drug conjugate and a VEGF signaling pathway inhibitor, wherein
preferably, the anti-HER3 antibody-drug conjugate is as defined in any one of claims 1-9, and the VEGF signaling pathway inhibitor is the anti-VEGF antibody as defined in any one of claims 5-7;
preferably, the tumor is non-small cell lung cancer; more preferably, the tumor is locally advanced or metastatic non-small cell lung cancer;
preferably, the tumor is an EGFR-mutant tumor; more preferably, the tumor is EGFR-mutant non-small cell lung cancer.

12. A pharmaceutical composition, comprising an anti-HER3 antibody-drug conjugate and a VEGF signaling pathway inhibitor, wherein
the anti-HER3 antibody-drug conjugate has a structure shown as the formula below: 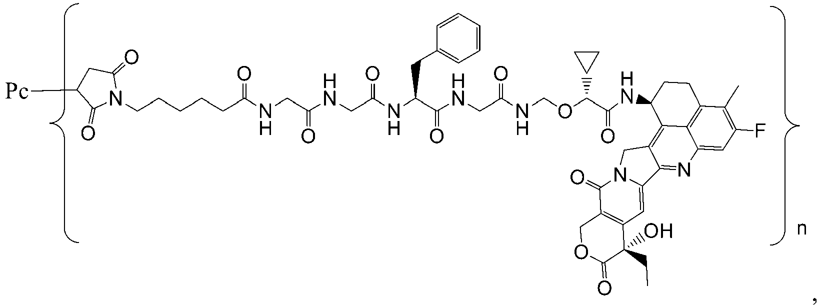
wherein n is 1-8, and Pc is an anti-HER3 antibody;
preferably, the VEGF signaling pathway inhibitor is preferably an anti-VEGF antibody;
preferably, the anti-HER3 antibody comprises a heavy chain variable region (VH) and a light chain variable region (VL), wherein the VH comprises a HCDR1, a HCDR2, and a HCDR3 set forth in SEQ ID NO: 1, SEQ ID NO: 2, and SEQ ID NO: 3, respectively, and the VL comprises a LCDR1, a LCDR2, and a LCDR3 set forth in SEQ ID NO: 4, SEQ ID NO: 5, and SEQ ID NO: 6, respectively; more preferably, the VH comprises the amino acid sequence set forth in SEQ ID NO: 7 or an amino acid sequence having at least 90% identity thereto, and the VL comprises the amino acid sequence set forth in SEQ ID NO: 8 or an amino acid sequence having at least 90% identity thereto;
more preferably, the anti-HER3 antibody comprises a heavy chain and a light chain, wherein the heavy chain comprises the amino acid sequence set forth in SEQ ID NO: 9 or an amino acid sequence having at least 90% identity thereto, and the light chain comprises the amino acid sequence set forth in SEQ ID NO: 10 or an amino acid sequence having at least 90% identity thereto;
preferably, the anti-VEGF antibody comprises a heavy chain variable region (VH) and a light chain variable region (VL), wherein the VH comprises a HCDR1, a HCDR2, and a HCDR3 set forth in SEQ ID NO: 11, SEQ ID NO: 12, and SEQ ID NO: 13, respectively, and the VL comprises a LCDR1, a LCDR2, and a LCDR3 set forth in SEQ ID NO: 14, SEQ ID NO: 15, and SEQ ID NO: 16, respectively;
more preferably, the anti-VEGF antibody comprises a heavy chain variable region (VH) and a light chain variable region (VL), wherein the VH comprises the amino acid sequence set forth in SEQ ID NO: 17 or an amino acid sequence having at least 90% identity thereto, and the VL comprises the amino acid sequence set forth in SEQ ID NO: 18 or an amino acid sequence having at least 90% identity thereto;
more preferably, the anti-VEGF antibody comprises a heavy chain and a light chain, wherein the heavy chain comprises the amino acid sequence set forth in SEQ ID NO: 19 or an amino acid sequence having at least 90% identity thereto, and the light chain comprises the amino acid sequence set forth in SEQ ID NO: 20 or an amino acid sequence having at least 90% identity thereto;
preferably, the pharmaceutical composition is used for treating a tumor, preferably for treating non-small cell lung cancer, and more preferably for treating locally advanced or metastatic non-small cell lung cancer; preferably, the tumor is an EGFR-mutant tumor; more preferably, the tumor is EGFR-mutant non-small cell lung cancer.

13. The pharmaceutical composition according to claim 12, wherein
i-1) the anti-HER3 antibody-drug conjugate is administered at a dose of about 0.1 mg/kg to about 20 mg/kg,
preferably about 1.0 mg/kg to about 20 mg/kg, about 1.0 mg/kg to about 13.5 mg/kg, about 1.0 mg/kg to about 12 mg/kg, about 1.5 mg/kg to about 10.5 mg, about 1.5 mg/kg to about 9.0 mg/kg; about 1.5 mg/kg to about 8.5 mg/kg, or about 1.5 mg/kg to about 8.0 mg/kg, and
more preferably about 1.0 mg/kg, about 1.5 mg/kg, about 3.0 mg/kg, about 4.5 mg/kg, about 5.0 mg/kg, about 5.5 mg/kg, about 6.0 mg/kg, about 6.5 mg/kg, about 7.0 mg/kg, about 7.5 mg/kg, about 8.0 mg/kg, about 8.5 mg/kg, about 9.0 mg/kg, about 10.5 mg/kg, about 12 mg/kg, or about 13.5 mg/kg;
i-2) the anti-HER3 antibody-drug conjugate is administered at a frequency of once every 1 week, once every 2 weeks, once every 3 weeks, once every 4 weeks, once every 6 weeks, once every 8 weeks, once every 10 weeks, or once every 12 weeks, preferably once every 2 weeks or once every 3 weeks;
and/or
ii-1) the VEGF signaling pathway inhibitor is administered at a dose of about 1.0 mg/kg to about 30 mg/kg,
preferably about 2.0 mg/kg to about 25 mg/kg, about 2.0 mg/kg to about 20 mg/kg, about 2.0 mg/kg to about 19 mg/kg, about 2.0 mg/kg to about 18 mg/kg, about 2.0 mg/kg to about 17 mg/kg, or about 2.0 mg/kg to about 16 mg/kg, and
more preferably about 5.0 mg/kg, about 6.0 mg/kg, about 6.5 mg/kg, about 7.0 mg/kg, about 7.5 mg/kg, 8.0 mg/kg, 8.5 mg/kg, about 9.0 mg/kg, about 9.5 mg/kg, about 10.0 mg/kg, about 10.5 mg/kg, about 11.0 mg/kg, about 11.5 mg/kg, about 12.0 mg/kg, about 12.5 mg/kg, about 13.0 mg/kg, about 13.5 mg/kg, about 14.0 mg/kg, about 14.5 mg/kg, about 15.0 mg/kg, about 15.5 mg/kg, or about 16.0 mg/kg;
ii-2) the VEGF signaling pathway inhibitor is administered at a frequency of once every 1 week, once every 2 weeks, once every 3 weeks, once every 4 weeks, once every 6 weeks, once every 8 weeks, once every 10 weeks, or once every 12 weeks, preferably once every 3 weeks.
